# EUROPEAN PATENT SPECIFICATION

(11) **EP 1 355 631 B1**
(45) Date of publication and mention of the grant of the patent: **03.10.2007**
(21) Application number: 01965984.6
(22) Date of filing: 17.08.2001
(51) Int. Cl.: A61K 9/20, A61K 31/635

(54) **ORAL FAST-MELT FORMULATION OF A CYCLOOXYGENASE-2 INHIBITOR**
IN DER MUNDHÖHLE SCHNELL ZERFALLENDE ORALE ARZNEIZUBEREITUNG ENTHALTEND EINEN CYCLOOXYGENASE-2 HEMMER
FORMULATION ORALE A DESINTEGRATION RAPIDE D'UN INHIBITEUR DE CYCLOOXYGENASE-2

(30) Priority: 18.08.2000 US 226347 P
(43) Date of publication of application: 29.10.2003
(73) Proprietor: Pharmacia Corporation, Peapack, NJ 07977 (US); Yamanouchi Pharma Technologies, Inc., Palo Alto, CA 94304 (US)
(72) Inventor: LE, Trang, T., Mundelein, IL 60060 (US); KARARLI, Tugrul, T., Skokie, IL 60076 (US); KONTNY, Mark, J., Libertyville, IL 60048 (US); SASTRY, Srikonda, V., Sunnyvale, CA 94087 (US); NYSHADHAM, Janaki, R., Fremont, CA 94539 (US); PAGLIERO, Arthur, J., Jr., Vacaville, CA 94303 (US)
(74) Representative: Motion, Keith Robert
(86) International application number: PCT/US2001/025803
(87) International publication number: WO 2002/015885

(56) References cited:
- EP-A- 0 745 382
- WO-A-00/32189
- WO-A-01/87264
- WO-A-99/47126
- SASTRY SV, NYSHADHAM JR, FIX JA: "Recent technological advances in oral drug delivery - a review" PHARMACEUTICAL SCIENCE & TECHNOLOGY TODAY, vol. 3, no. 4, April 2000 (2000-04), pages 138-145, XP002196787

## Description

### FIELD OF THE INVENTION

The present invention relates to orally deliverable pharmaceutical compositions containing a selective cyclooxygenase-2 inhibitory drug, to processes for preparing such compositions, and to the use of such compositions in the manufacture of medicaments.

### BACKGROUND OF THE INVENTION

Numerous compounds have been reported having therapeutically and/or prophylactically useful selective cyclooxygenase-2 inhibitory effect, and have been disclosed as having utility in treatment or prevention of specific cyclooxygenase-2 mediated disorders or of such disorders in general. Among such compounds are a large number of substituted pyrazolyl benzenesulfonamides as reported in U.S. Patent No. 5,760,068 to Talley et al.*,* including for example the compound 4-[5-(4-methylphenyl)-3-(trifluoromethyl)-1H-pyrazol-1-yl]benzenesulfonamide, also referred to herein as celecoxib (I), and the compound 4-[5-(3-fluoro-4-methoxyphenyl)-3-difluoromethyl)-1H-pyrazol-1-yl]benzenesulfonamide, also referred to herein as deracoxib (II).

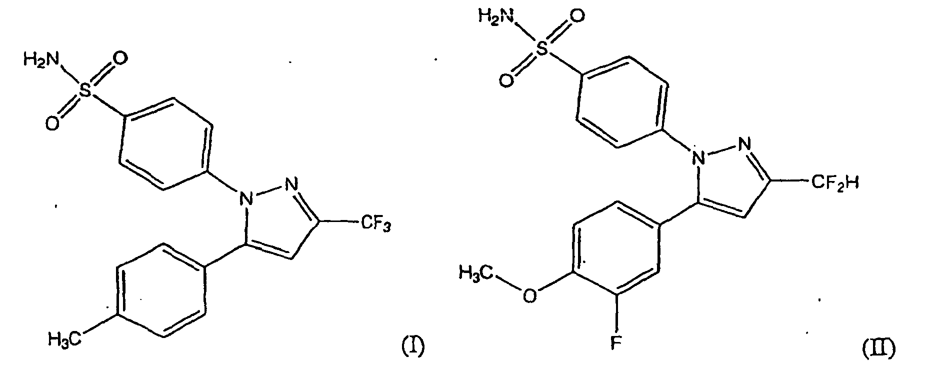

Other compounds reported to have therapeutically and/or prophylactically useful selective cyclooxygenase-2 inhibitory effect are substituted isoxazolyl benzenesulfonamides as reported in U.S. Patent No. 5,633,272 to Talley et al., including for example the compound 4-[5-methyl-3-phenylisoxazol-4-yl]benzenesulfonamide, also referred to herein as valdecoxib (III).

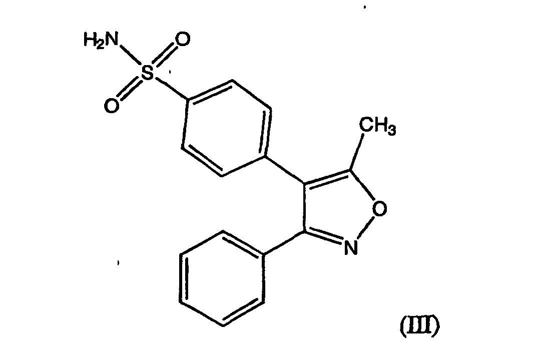

Still other compounds reported to have therapeutically and/or prophylactically useful selective cyclooxygenase-2 inhibitory effect are substituted (methylsulfonyl)phenyl furanones as reported in U.S. Patent No. 5,474,995 to Ducharme et al.*,* including for example the compound 3-phenyl-4-[4-(methylsulfonyl)phenyl]-5H-furan-2-one, also referred to herein as rofecoxib (IV).

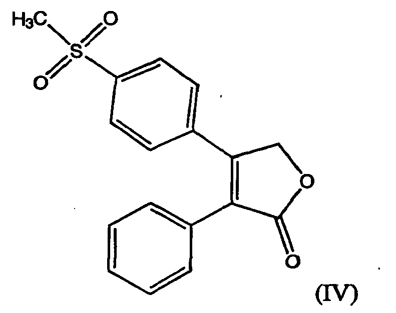

U.S. Patent No. 5,981,576 to Belley et al. discloses a further series of (methylsulfonyl)phenyl furanones said to be useful as selective cyclooxygenase-2 inhibitory drugs, including 3-(1-cyclopropylmethoxy)-5,5-dimethyl-4-[4-(methylsulfonyl)phenyl]-5H-furan-2-one and 3-(1-cyclopropylethoxy)-5,5-dimethyl-4-[4-(methylsulfonyl)phenyl]-5H-furan-2-one.

U.S. Patent No. 5,861,419 to Dube et al. discloses substituted pyridines said to be useful as selective cyclooxygenase-2 inhibitory drugs, including for example the compound 5-chloro-3-(4-methylsulfonyl)phenyl-2-(2-methyl-5-pyridinyl)pyridine, also referred to herein as etoricoxib (V).

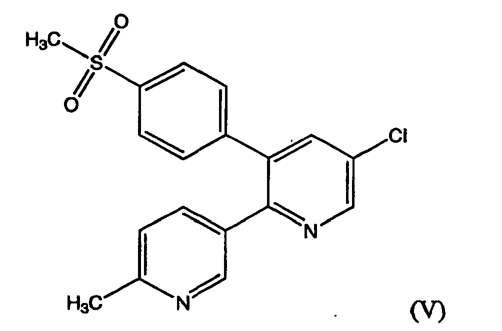

European Patent Application No. 0 863 134 discloses the compound 2-(3,5-difluorophenyl)-3-[4-(methylsulfonyl)phenyl]-2-cyclopenten-1-one said to be useful as a selective cyclooxygenase-2 inhibitory drug.

U.S. Patent No. 6,034,256 to Carter et al. discloses a series of benzopyrans said to be useful as selective cyclooxygenase-2 inhibitory drugs, including the compound (S)-6,8-dichloro-2-(trifluoromethyl)-2H-1-benzopyran-3-carboxylic acid (VI).

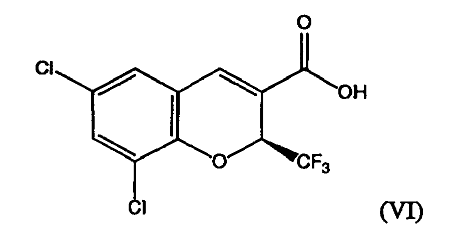

International Patent Publication No. WO 00/24719 discloses substituted pyridazinones said to be useful as selective cyclooxygenase-2 inhibitory drugs, including the compound 2-(3,4-difluorophenyl)-4-(3-hydroxy-3-methyl-1-butoxy)-5-[4-(methylsulfonyl)phenyl]-3-(2H)-pyridazinone.

A need for formulated compositions of selective cyclooxygenase-2 inhibitory drugs, in particular, easy-to-swallow compositions, exists. Easy-to-swallow drug delivery systems can provide many benefits over conventional dosage forms, particularly to populations such as the elderly, young children and other groups of patients that have difficulty swallowing conventional oral preparations.

Common oral dosage forms such as tablets, pills or capsules generally must be swallowed with water. Many pediatric and elderly patients with weak swallowing ability are unwilling or unable to swallow such dosage forms.

Powders and granules are additional commonly used oral dosage forms. However, these formulations can be difficult to swallow completely due to their tendency to remain in the oral cavity. In some instances, patients taking powdered dosage forms will feel choked with powder or feel pain or unpleasantness due to granules being lodged under dentures. Additionally, powders and granules typically can only be used after the tearing or breaking of a package, tasks that elderly patients often find difficult to perform.

Further, powder and granule dosage forms are inconvenient to take as they typically must be diluted with a suitable amount of water or other liquid carrier prior to ingestion. This is particularly problematic when the medication is needed to provide fast relief of pain, since water is not always readily obtainable throughout the day. Moreover, powders or granules taken after dissolution or suspension in a liquid can also be difficult for elderly patients suffering from incontinence as such patients may experience urination problems at night when relatively large volumes of liquid-based medications are taken before bedtime.

Syrups and elixirs are additional commonly used oral dosage forms. However, elderly patients and others who have difficulty in measuring precise volumes are unlikely to be able to administer to themselves a proper dose and therefore require assistance at each administration.

International Patent Publication No. WO 00/32189 discloses various oral preparations of celecoxib. However, easy-to-swallow solid preparations of compositions containing selective cyclooxygenase-2 inhibitory drugs have not been disclosed.

In light of the expanding elderly population, it is becoming critically important to develop safe, effective, easy-to-swallow pharmaceutical preparations to treat age-related indications, wherein such preparations are convenient for elderly patients to self-administer and ingest.

U.S. Patent No. 5,576,014 discloses an intrabuccally dissolving compressed molding prepared by granulating together a low moldability saccharide with a high moldability saccharide and compressing the granulate into a molding. The resulting molding can incorporate a drug and is said to show quick disintegration and dissolution in the buccal cavity but to maintain sufficient hardness so as not break during production and distribution. The compressed molding of U.S. Patent No. 5,576,014 is a type of dosage form known as a "fast-melt tablet", exhibiting rapid disintegration, usually associated with the carrier materials, typically sugars, and concomitant rapid dissolution or dispersion of the drug in the mouth, usually without need for water other than that contained in saliva. A drug formulated in such a tablet is readily swallowed.

International Patent Publication No. WO 99/47126 describes rapidly disintegrating tablets comprising ibuprofen (20% by weight), mannitol (85% by weight), maltose (5% by weight) and polyvinylpyrrolidone (0.5% by weight), made by wet granulation.

However, selective cyclooxygenase-2 inhibitory drugs present certain challenges for formulation as fast-melt tablets. For example, many selective cyclooxygenase-2 inhibitory compounds, including celecoxib, deracoxib, valdecoxib, 2-(3,5-difluorophenyl)-3-[4-(methylsulfonyl)phenyl]-2-cyclopenten-1-one, etoricoxib and rofecoxib, have very low solubility in aqueous media. In addition, some, for example celecoxib, have relatively high dose requirements. Celecoxib also presents difficulties as a result of unique physical and chemical characteristics such as electrostatic and cohesive properties, low bulk density, low compressibility and poor flow properties.

Due at least in part to these properties, celecoxib crystals tend to segregate and agglomerate together during mixing, resulting in a non-uniformly blended composition containing undesirably large, insoluble aggregates of celecoxib. Therefore, it is difficult to prepare a fast-melt composition containing celecoxib that has the desired blend uniformity for rapid and complete disintegration in the mouth.

The term "fast-melt" as used herein refers to a composition such as a tablet wherein an active agent or drug is distributed or dispersed in a matrix formed by a carrier that, upon oral administration to a subject, disintegrates in the oral cavity, thereby releasing the drug, typically in particulate form, for entry to the gastrointestinal tract by swallowing, and subsequent absorption. The term "oral cavity" includes the entire interior of the mouth, including not only the buccal cavity (that part of the oral cavity anterior to the teeth and gums) but also the sublingual and supralingual spaces.

With respect to drugs requiring a high dose for therapeutic effectiveness, the large size of a fast-melt tablet required to provide a therapeutic dose may be a limiting factor. To reduce tablet size, drug loading can be increased in a given formulation. However, typical fast-melt tablet formulations begin to lose their rapid disintegration characteristics as the relative amount of active agent in the tablet increases, at least in part because of the corresponding reduction in the amount of readily soluble and/or disintegratable carrier. Alternatively, several tablets having a low drug loading would have to be ingested, which can result in patient inconvenience and decreased compliance.

The physical and chemical characteristics of celecoxib described above also present challenges during granulation. In order to be compressed as tablets, drugs with poor flow and/or compressibility characteristics such as celecoxib must normally be granulated by a wet granulation method prior to tableting, for example by high-shear granulation or by fluid bed granulation.

The term "granulation fluid" as used herein refers to any liquid material that is added to a powder bed, for example by spraying, during fluid bed granulation or other wet granulation processes. The granulation fluid can, but is not required to, contain a binder and/or other excipients either in solution or in suspension. The term "powder bed" as used herein refers to all material including solid particulates and granulation fluid added thereto that is present in a granulation bowl at any point during wet granulation.

Fluid bed granulation provides several advantages over traditional high-shear wet granulation. For example, the handling required and the potential for contamination by dust are reduced. Fluid bed granulation also can be more readily automated than high-shear granulation and offers savings of both handling time and space. However, the above-mentioned challenges presented by celecoxib and other cyclooxygenase-2 inhibitory drugs of low water solubility can make fluid bed granulation difficult, particularly as drug loading increases. For example, celecoxib particles have an inherently electrostatic, cohesive nature that promotes agglomeration of the particles. Further, the highly water-insoluble, hydrophobic nature of celecoxib inhibits wetting of these agglomerated drug particles by the granulation fluid during granulation. This lack of wetting inhibits separation of the drug agglomerates. During the process of fluid bed granulation, such agglomeration and poor wetting can act to prevent complete fluidization of the material being granulated and ultimately lead to ineffective granulation.

Ineffective granulation can, among other effects, lead to formulation of nonuniform tablets which have low crushing strength and/or increased disintegration time, characteristics which are highly undesirable for fast-melt tablets. Tablets with low crushing strength tend to break upon removal from blister packages or, alternatively, are too soft to maintain their individual integrity when bottled.

For these and other reasons, therefore, if some of the difficulties discussed above could be overcome, it would be a much desired advance in the art to provide a fast-melt formulation of a selective cyclooxygenase-2 inhibitory drug of low solubility, such as celecoxib, that can be produced by a conventional wet granulation process.

### SUMMARY OF THE INVENTION

According to the present invention, there is now provided the process of claim 1.

The term "low moldability" as applied to a saccharide herein refers, in accordance with above-cited U.S. Patent No. 5,576,014, to a saccharide which generally shows a hardness of less than 19.6 N (2 kp) when 150 mg of the saccharide is made into a tablet using a punch of 8 mm in diameter under a pressure of 10 to 50 kg/cm². Thus a saccharide of low moldability as required herein can be, for example, a "non-direct compression sugar" as defined in U.S. Patent No. 6,024,981 to Khankari et al.

In particular, a saccharide of low moldability typically has a fine particle size, for example an average particle size of 10 µm to 80 µm, and is not pre-granulated. Above-cited U.S. Patent No. 6,024,981 discloses that it is well known in the pharmaceutical industry that decreasing the particle size of a sugar decreases its compressibility and fluidity. Not all saccharides, even at fine particle size, are "non-direct compression sugars" or saccharides of low moldability as required herein. Examples of saccharides of low moldability, at least when in finely particulate form without pre-granulation, include lactose, mannitol, glucose, sucrose, xylitol, *etc.*

The term "high moldability" as applied to a saccharide herein refers, in accordance with above-cited U.S. Patent No. 5,576,014, to a saccharide which generally shows a hardness of 19.6 N (2 kp) or more when 150 mg of the saccharide is made into a tablet using a punch of 8 mm in diameter under a pressure of 10 to 50 kg/cm². Thus a saccharide of high moldability as required herein can be, for example, a "direct compression sugar" as defined in above-cited U.S. Patent No. 6,024,981. Examples of saccharides of high moldability include maltose, maltitol, sorbitol, *etc*.

The means to inhibit agglomeration incorporated in a process of the invention is any measure taken during production of the fast-melt composition to prevent or reduce drug agglomeration or to facilitate separation of existing drug agglomerates. For example, in fluid bed granulation, means to inhibit agglomeration can include addition of a wetting agent, having the effect of providing improved wetting by the granulation fluid of the powder material to be granulated. Alternatively or in addition, means to inhibit agglomeration during granulation can include, for example, pre-wetting the powder material to be granulated, such as by employing an additional, external processor with spraying capacity; and/or using an air distributor plate adapted to increase air flow along the periphery of the granulation bowl.

Inhibition of agglomeration can be achieved by various mechanisms. The particular mechanism is not critical so long as the end result of reduced agglomeration and/or separation of agglomerates is achieved.

In a process of the invention, the selective cyclooxygenase-2 inhibitory drug, the saccharide of low moldability and, optionally, other excipients can, if desired, be separately wet granulated. However, in a presently preferred embodiment, a process of the invention comprises wet granulation of a selective cyclooxygenase-2 inhibitory drug together with a saccharide of low moldability and a saccharide of high moldability, more preferably also together with a wetting agent.

In another preferred embodiment, a process of the invention comprises a step of blending a selective cyclooxygenase-2 inhibitory drug with a saccharide of low moldability, followed by a step of wet granulating the resulting blend with a saccharide having high moldability and a wetting agent.

In still another preferred embodiment, a process of the invention comprises wet granulation of a saccharide having low moldability with a saccharide having high moldability to obtain granules of a first kind, wet granulation of a selective cyclooxygenase-2 inhibitory drug together with a saccharide having high moldability and a wetting agent to obtain granules of a second kind, and admixing the granules of said first and second kinds.

An especially preferred process of the invention further comprises a step of compressing the wet granulated, for example fluid bed granulated, composition prepared by any of the processes summarized above to produce a solid dosage form, for example an oral fast-melt tablet.

An oral fast-melt composition as defined in claim 23, having a selective cyclooxygenase-2 inhibitory drug dispersed in a matrix comprising a saccharide of low moldability and a saccharide of high moldability, is a further embodiment of the present invention. A preferred composition of this embodiment is an oral fast-melt tablet. A still further embodiment is an oral fast-melt composition, for example, an oral fast-melt tablet, prepared by a process as herein described.

Tablets of the invention disintegrate within 30 to 150 seconds after placement in a standard *in vitro* disintegration assay (conducted according to U.S. Pharmacopeia 24 (2000), Test No. 701). Preferably, they disintegrate within 5 to 60 seconds after placement in the oral cavity of a subject. Preferably, such tablets have a hardness of 9.8 N to 98 N (1 kp to 10 kp).

In a particularly preferred embodiment of the invention, an oral fast-melt pharmaceutical composition is provided, comprising a selective cyclooxygenase-2 inhibitory drug of low water solubility dispersed in a matrix comprising a saccharide of low moldability, a saccharide of high moldability and a wetting agent. Such a composition can be prepared by a process herein described or by any known process. The term "dispersed" in the present context means that the drug is substantially non-agglomerated. The wetting agent is present in an amount sufficient to inhibit agglomeration of the drug during preparation of the composition. Compositions of this embodiment are preferably tablets having disintegration and hardness properties as defined above.

Processes of the present invention have been found to resolve at least some of the difficulties alluded to above in a surprisingly effective manner. Thus, in a significant advance in the art, a selective cyclooxygenase-2 inhibitory drug of low water solubility is now presented in a novel, easy-to-swallow, fast-melt formulation. A particular advantage of processes of the invention is that oral fast-melt tablets containing a cyclooxygenase-2 inhibitory drug of low water solubility, even such a drug having a relatively high dosage requirement, for example celecoxib, can be prepared by fluid bed granulation and compression. These oral fast-melt tablets provide a heretofore nonexistent dosage form of a selective cyclooxygenase-2 inhibitory drug that is efficient to produce, convenient and easy to swallow.

Also provided by the present invention is a method of use of a composition of the invention for preparing a medicament. Other features of this invention will be in part apparent and in part pointed out hereinafter.

### DETAILED DESCRIPTION OF THE INVENTION

The present invention provides a process for preparing an intraorally disintegrating pharmaceutical composition, *i.e*., an oral fast-melt composition, of a selective cyclooxygenase-2 inhibitory drug. The process comprises a step of wet granulating the drug together with a binding agent comprising a saccharide of high moldability, and a step of blending with the drug a saccharide of low moldability. The wet granulating step and the blending step can take place in any order or simultaneously to form granules. The process incorporates means to inhibit agglomeration of the drug. Compositions prepared by such a process represent an embodiment of the present invention.

A further embodiment of the invention is an intraorally disintegrating pharmaceutical composition as defined in claim 23, comprising a selective cyclooxygenase-2 inhibitory drug of low water solubility dispersed in a matrix comprising a saccharide having low moldability, a saccharide having high moldability and, preferably, a wetting agent, the wetting agent being present in an amount sufficient to inhibit agglomeration of the drug during preparation of the composition.

A further embodiment of the invention is an intraorally disintegrating pharmaceutical composition as defined in claim 23, comprising a selective cyclooxygenase-2, inhibitory drug of low water solubility dispersed in a matrix comprising a saccharide having low moldability, a saccharide having high moldability, and a glidant, preferably silicon dioxide. Such a composition can further comprise a wetting agent.

Processes and compositions of the invention are especially useful for selective cyclooxygenase-2 inhibitory compounds as defined in claim 1, having solubility in water lower than about 1 mg/ml. In particular, processes and compositions of the invention are used for compounds having the formula (VII): 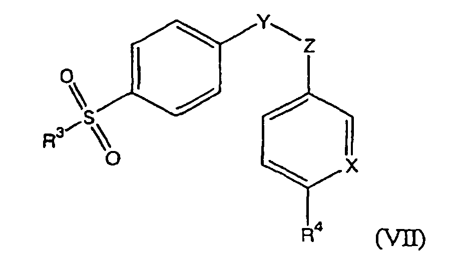 where R³ is a methyl or amino group, R⁴ is hydrogen or a C₁₋₄ alkyl or alkoxy group, X is N or CR⁵ where R⁵ is hydrogen or halogen, and Y and Z are independently carbon or nitrogen atoms defining adjacent atoms of a five- to six-membered ring that is unsubstituted or substituted at one or more positions with oxo, halo, methyl or halomethyl groups. Preferred such five- to six-membered rings are cyclopentenone, furanone, methylpyrazole, isoxazole and pyridine rings substituted at no more than one position.

Illustratively, processes and compositions of the invention are suitable for celecoxib, deracoxib, valdecoxib, rofecoxib, etoricoxib, 2-(3,5-difluorophenyl)-3-[4-(methylsulfonyl)phenyl]-2-cyclopenten-1-one, (S)-6,8-dichloro-2-(trifluoromethyl)-2H-1-benzopyran-3-carboxylic acid and 2-(3,4-difluorophenyl)-4-(3-hydroxy-3-methyl-1-butoxy)-5-[4-(methylsulfonyl)phenyl]-3-(2H)-pyridazinone, more particularly celecoxib, valdecoxib, rofecoxib and etoricoxib, and still more particularly celecoxib and valdecoxib.

The invention is illustrated herein with particular reference to celecoxib, and it will be understood that any other selective cyclooxygenase-2 inhibitory compound of low solubility in water can, if desired, be substituted in whole or in part for celecoxib in processes and compositions herein described.

### Preparation of a selective cyclooxrygenase-2 inhibitory drug

Celecoxib used in the process and compositions of the present invention can be prepared by a process known *per se*, for example by processes set forth in U.S. Patent No. 5,466,823 to Talley et al. or in U.S. Patent No. 5,892,053 to Zhi & Newaz.

Other selective cyclooxygenase-2 inhibitory drugs can be prepared by processes known *per se*, including processes set forth in patent publications disclosing such drugs; for example in the case of valdecoxib in above-cited U.S. Patent No. 5,633,272, and in the case of rofecoxib in above-cited U.S. Patent No. 5,474,995.

### Dosage provided by compositions of the invention

Celecoxib compositions of the present invention preferably comprise celecoxib in a daily dosage amount of 10 mg to 1000 mg, more preferably 25 mg to 400 mg, and most preferably 50 mg to 200 mg.

For other selective cyclooxygenase-2 inhibitory drugs, a daily dosage amount can be in a range known to be therapeutically effective for such drugs. Preferably, the daily dosage amount is in a range providing therapeutic equivalence to celecoxib in the daily dosage ranges indicated immediately above.

Dosage units of celecoxib compositions of the invention typically contain 10 mg to 400 mg of celecoxib, for example, a 10, 20, 37.5, 50, 75, 100, 125, 150, 175, 200, 250, 300,350 or 400 mg dose of celecoxib. Preferred dosage units contain 25 mg to 400 mg of celecoxib. More preferred dosage unit forms contain 50 mg to 200 mg of celecoxib. A particular dosage unit can be selected to accommodate the desired frequency of administration used to achieve a specified daily dosage. The amount of the unit dosage form of the composition that is administered and the dosage regimen for treating the condition or disorder will depend on a variety of factors, including the age, weight, sex and medical condition of the subject, the severity of the condition or disorder, the route and frequency of administration, and the particular selective cyclooxygenase-2 Inhibitory drug selected, and thus may vary widely. It is contemplated, however, that for most purposes a once-a-day or twice-a-day administration regimen provides the desired therapeutic efficacy.

In a celecoxib composition, celecoxib can be present in the composition at a minimum concentration of about 1%, preferably about 4%, more preferably about 10%, and still more preferably about 20%, by weight. Where the selective cyclooxygenase-2 inhibitory drug is therapeutically effective at lower dosages than celecoxib, the minimum concentration can be lower than that indicated immediately above for celecoxib; for example in the case of valdecoxib the drug can be present at a minimum concentration of about 0.1 % by weight. Celecoxib can be present in the composition at a maximum concentration of about 60%, more typically about 50%, by weight.

### Utility of compositions of the invention

Compositions of the present invention are useful in treatment and prevention of a very wide range of disorders mediated by cyclooxygenase-2 (COX-2), including but not restricted to disorders characterized by inflammation, pain and/or fever. Such compositions are especially useful as anti-inflammatory agents, such as in treatment of arthritis, with the additional benefit of having significantly less harmful side effects than compositions of conventional nonsteroidal anti-inflammatory drugs (NSAIDs) that lack selectivity for COX-2 over COX-1. In particular, such compositions have reduced potential for gastrointestinal toxicity and gastrointestinal irritation including upper gastrointestinal ulceration and bleeding, reduced potential for renal side effects such as reduction in renal function leading to fluid retention and exacerbation of hypertension, reduced effect on bleeding times including inhibition of platelet function, and possibly a lessened ability to induce asthma attacks in aspirin-sensitive asthmatic subjects, by comparison with compositions of conventional NSAIDs. Thus compositions of the invention comprising a selective COX-2 inhibitory drug are particularly useful as an alternative to conventional NSAIDs where such NSAIDs are contraindicated, for example in patients with peptic ulcers, gastritis, regional enteritis, ulcerative colitis, diverticulitis or with a recurrent history of gastrointestinal lesions; gastrointestinal bleeding, coagulation disorders including anemia such as hypoprothrombinemia, hemophilia or other bleeding problems; kidney disease; or in patients prior to surgery or patients taking anticoagulants.

Such compositions are useful to treat arthritic disorders, including but not limited to rheumatoid arthritis, spondyloarthropathies, gouty arthritis, osteoarthritis, systemic lupus erythematosus and juvenile arthritis.

Such compositions are also useful in treatment of asthma, bronchitis, menstrual cramps, preterm labor, tendinitis, bursitis, allergic neuritis, cytomegalovirus infectivity, apoptosis including HIV-induced apoptosis, lumbago, liver disease including hepatitis, skin-related conditions such as psoriasis, eczema, acne, burns, dermatitis and ultraviolet radiation damage including sunburn, and post-operative inflammation including that following ophthalmic surgery such as cataract surgery or refractive surgery.

Such compositions are useful to treat gastrointestinal conditions such as inflammatory bowel disease, Crohn's disease, gastritis, irritable bowel syndrome and ulcerative colitis.

Such compositions are useful in treating inflammation in such diseases as migraine headaches, periarteritis nodosa, thyroiditis, aplastic anemia, Hodgkin's disease, sclerodoma, rheumatic fever, type I diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury including brain edema, myocardial ischemia, and the like.

Such compositions are useful in treatment of ophthalmic diseases, such as retinitis, scleritis, episcleritis, conjunctivitis, retinopathies, uveitis, ocular photophobia, and of acute injury to eye tissue.

Such compositions are useful in treatment of pulmonary inflammation, such as that associated with viral infections and cystic fibrosis, and in bone resorption such as that associated with osteoporosis.

Such compositions are useful for treatment of certain central nervous system disorders, such as cortical dementias including Alzheimer's disease, neurodegeneration, and central nervous system damage resulting from stroke, ischemia and trauma. The term "treatment" in the present context includes partial or total inhibition of dementias, including Alzheimer's disease, vascular dementia, multi-infarct dementia, pre-senile dementia, alcoholic dementia and senile dementia.

Such compositions are useful in treatment of allergic rhinitis, respiratory distress syndrome, endotoxin shock syndrome and liver disease.

Such compositions are useful in treatment of pain, including but not limited to postoperative pain, dental pain, muscular pain, and pain resulting from cancer. For example, such compositions are useful for relief of pain, fever and inflammation in a variety of conditions including rheumatic fever, influenza and other viral infections including common cold, low back and neck pain, dysmenorrhea, headache, toothache, sprains and strains, myositis, neuralgia, synovitis, arthritis, including rheumatoid arthritis, degenerative joint diseases (osteoarthritis), gout and ankylosing spondylitis, bursitis, burns, and trauma following surgical and dental procedures.

Such compositions are useful for, but not limited to, treating and preventing inflammation-related cardiovascular disorders in a subject. Such compositions are useful for treatment and prevention of vascular diseases, coronary artery disease, aneurysm, vascular rejection, arteriosclerosis, atherosclerosis including cardiac transplant atherosclerosis, myocardial infarction, embolism, stroke, thrombosis including venous thrombosis, angina including unstable angina, coronary plaque inflammation, bacterial-induced inflammation including Chlamydia-induced inflammation, viral induced inflammation, and inflammation associated with surgical procedures such as vascular grafting including coronary artery bypass surgery, revascularization procedures including angioplasty, stent placement, endarterectomy, or other invasive procedures involving arteries, veins and capillaries.

Such compositions are useful for, but not limited to, treatment of angiogenesis-related disorders in a subject, for example to inhibit tumor angiogenesis. Such compositions are useful for treatment of neoplasia, including metastasis; ophthalmological conditions such as corneal graft rejection, ocular neovascularization, retinal neovascularization including neovascularization following injury or infection, diabetic retinopathy, macular degeneration, retrolental fibroplasia and glaucoma, including neovascular glaucoma; ulcerative diseases such as gastric ulcer, pathological, but non-malignant, conditions such as hemangiomas, including infantile hemangiomas, angiofibroma of the nasopharynx and avascular necrosis of bone; and disorders of the female reproductive system such as endometriosis.

Such compositions are useful for prevention or treatment of benign and malignant tumors/neoplasia including cancers, for example colorectal cancer, brain cancer, bone cancer, epithelial cell-derived neoplasia (epithelial carcinoma) such as basal cell carcinoma, adenocarcinoma, gastrointestinal cancer such as lip cancer, mouth cancer, esophageal cancer, small bowel cancer, stomach cancer, colon cancer, liver cancer, bladder cancer, pancreas cancer, ovary cancer, cervical cancer, lung cancer, breast cancer and skin cancer, such as squamous cell and basal cell cancers, prostate cancer, renal cell carcinoma, and other known cancers that affect epithelial cells throughout the body. Neoplasias for treatment of which compositions of the invention are contemplated to be particularly useful are gastrointestinal cancer, Barrett's esophagus, liver cancer, bladder cancer, pancreas cancer, ovary cancer, prostate cancer, cervical cancer, lung cancer, breast cancer and skin cancer, such as squamous cell and basal cell cancers. Compositions of the invention can also be used to treat fibrosis that occurs with radiation therapy. Such compositions can be used to treat subjects having adenomatous polyps, including those with familial adenomatous polyposis (FAP). Additionally, such compositions can be used to prevent polyps from forming in patients at risk of FAP.

Such compositions inhibit prostanoid-induced smooth muscle contraction by preventing synthesis of contractile prostanoids and hence can be of use in treatment of dysmenorrhea, premature labor, asthma and eosinophil-related disorders. They also can be of use for decreasing bone loss particularly in postmenopausal women (*i*.*e*., treatment of osteoporosis), and for treatment of glaucoma.

Preferred uses for compositions of the present invention are for treatment of rheumatoid arthritis and osteoarthritis, for pain management generally (particularly post-oral surgery pain, post-general surgery pain, post-orthopedic surgery pain, and acute flares of osteoarthritis), for treatment of Alzheimer's disease, and for colon cancer chemoprevention.

For treatment of rheumatoid arthritis or osteoarthritis, such compositions of the invention can be used to provide a daily dosage of celecoxib of 50 mg to 1000 mg, preferably 100 mg to 600 mg, more preferably 150 mg to 500 mg, still more preferably 175 mg to 400 mg, for example about 200 mg. A daily dose of celecoxib of 0.7 to 13 mg/kg body weight, preferably 1.3 to 8 mg/kg body weight, more preferably 2 to 6.7 mg/kg body weight, and still more preferably 2.3 to 5.3 mg/kg body weight, for example about 2.7 mg/kg body weight, is generally appropriate when administered in a composition of the invention. The daily dose can be administered in one to about four doses per day, preferably one or two doses per day.

For treatment of Alzheimer's disease or cancer, such compositions of the invention can be used to provide a daily dosage of celecoxib of 50 mg to 1000 mg, preferably 100 mg to 800 mg, more preferably 150 mg to 600 mg, and still more preferably 175 mg to 400 mg, for example about 400 mg. A daily dose of 0.7 to 13 mg/kg body weight, preferably

1.3 to 10.7 mg/kg body weight, more preferably 2 to 8 mg/kg body weight, and still more preferably 2.3 to 5.3 mg/kg body weight, for example about 5.3 mg/kg body weight, is generally appropriate when administered in a composition of the invention. The daily dose can be administered in one to about four doses per day, preferably one or two doses per day.

For pain management generally and specifically for treatment and prevention of headache and migraine, such compositions of the invention can be used to provide a daily dosage of celecoxib of 50 mg to 1000 mg, preferably 100 mg to 600 mg, more preferably 150 mg to 500 mg, and still more preferably 175 mg to . 400 mg, for example about 200 mg. A daily dose of celecoxib of 0.7 to 13 mg/kg body weight, preferably 1.3 to 8 mg/kg body weight, more preferably 2 to 6.7 mg/kg body weight, and still more preferably 2.3 to 5.3 mg/kg body weight, for example about 2.7 mg/kg body weight, is generally appropriate when administered in a composition of the invention. The daily dose can be administered in one to about four doses per day. Administration at a rate of one 50 mg dose unit four times a day, one 100 mg dose unit or two 50 mg dose units twice a day or one 200 mg dose unit, two 100 mg dose units or four 50 mg dose units once a day is preferred.

For selective cyclooxygenase-2 inhibitory drugs other than celecoxib, appropriate doses can be selected by reference to the patent literature cited hereinabove.

Besides being useful for human treatment, compositions of the invention are also useful for veterinary treatment of companion animals, exotic animals, farm animals, and the like, particularly mammals including rodents. More particularly, compositions of the invention are useful for veterinary treatment of cyclooxygenase-2 mediated disorders in horses, dogs and cats.

### Method of treatment

The present invention also relates to a therapeutic method (not claimed as such) of treating a condition or disorder where treatment with a cyclooxygenase-2 inhibitory drug is indicated, the method comprising oral administration of one or more pharmaceutical compositions of the present invention to a patient in need thereof. The dosage regimen to prevent, give relief from, or ameliorate the condition or disorder preferably corresponds to once-a-day or twice-a-day treatment, but can be modified in accordance with a variety of factors. These include the type, age, weight, sex, diet and medical condition of the patient and the nature and severity of the disorder. Thus, the dosage regimen actually employed can vary widely and can therefore deviate from the preferred dosage regimens set forth above.

Initial treatment of a patient suffering from a condition or disorder where treatment with a cyclooxygenase-2 inhibitory drug is indicated can begin with a dose regimen as indicated above. Treatment is generally continued as necessary over a period of several weeks to several months or years until the condition or disorder has been controlled or eliminated. Patients undergoing treatment with a composition of the invention can be routinely monitored by any of the methods well known in the art to determine the effectiveness of therapy. Continuous analysis of data from such monitoring permits modification of the treatment regimen during therapy so that optimally effective amounts of the drug are administered at any point in time, and so that the duration of treatment can be determined. In this way, the treatment regimen and dosing schedule can be rationally modified over the course of therapy so that the lowest amount of the drug exhibiting satisfactory effectiveness is administered, and so that administration is continued only for so long as is necessary to successfully treat the condition or disorder.

The present compositions can be used in combination therapies with opioids and other analgesics, including narcotic analgesics, Mu receptor antagonists, Kappa receptor antagonists, non-narcotic (i.e. non-addictive) analgesics, monamine uptake inhibitors, adenosine regulating agents, cannabinoid derivatives, Substance P antagonists, neurokinin-1 receptor antagonists and sodium channel blockers, among others. Preferred combination therapies comprise use of a composition of the invention with one or more compounds selected from aceclofenac, acemetacin, *e*-acetamidocaproic acid, acetaminophen, acetaminosalol, acetanilide, acetylsalicylic acid (aspirin), *S*-adenosylmethionine, alclofenac, alfentanil, allylprodine, alminoprofen, aloxiprin, alphaprodine, aluminum bis(acetylsalicylate), amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antipyrine salicylate, antrafenine, apazone, bendazac, benorylate, benoxaprofen, benzpiperylon, benzydamine, benzylmorphine, bermoprofen, bezitramide, α-bisabolol, bromfenac, *p*-bromoacetanilide, 5-bromosalicylic acid acetate, bromosaligenin, bucetin, bucloxic acid, bucolome, bufexamac, bumadizon, buprenorphine, butacetin, butibufen, butophanol, calcium acetylsalicylate, carbamazepine, carbiphene, carprofen, carsalam, chlorobutanol, chlorthenoxazin, choline salicylate, cinchophen, cinmetacin, ciramadol, clidanac, clometacin, clonitazene, clonixin, clopirac, clove, codeine, codeine methyl bromide, codeine phosphate, codeine sulfate, cropropamide, crotethamide, desomorphine, dexoxadrol, dextromoramide, dezocine, diampromide, diclofenac sodium, difenamizole, difenpiramide, diflunisal, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, flufenamic acid, flunoxaprofen, fluoresone, flupirtine, fluproquazone, flurbiprofen, fosfosal, gentisic acid, glafenine, glucametacin, glycol salicylate, guaiazulene, hydrocodone, hydromorphone, hydroxypethidine, ibufenac, ibuprofen, ibuproxam, imidazole salicylate, indomethacin, indoprofen, isofezolac, isoladol, isomethadone, isonixin, isoxepac, isoxicam, ketobemidone, ketoprofen, ketorolac, *p*-lactophenetide, lefetamine, levorphanol, lofentanil, lonazolac, lomoxicam, loxoprofen, lysine acetylsalicylate, magnesium acetylsalicylate, meclofenamic acid, mefenamic acid, meperidine, meptazinol, mesalamine, metazocine, methadone hydrochloride, methotrimeprazine, metiazinic acid, metofoline, metopon, mofebutazone, mofezolac, morazone, morphine, morphine hydrochloride, morphine sulfate, morpholine salicylate, myrophine, nabumetone, nalbuphine, 1-naphthyl salicylate, naproxen, narceine, nefopam, nicomorphine, nifenazone, niflumic acid, nimesulide, 5'-nitro-2'-propoxyacetanilide, norlevorphanol, normethadone, normorphine, norpipanone, olsalazine, opium, oxaceprol, oxametacine, oxaprozin, oxycodone, oxymorphone, oxyphenbutazone, papaveretum, paranyline, parsalmide, pentazocine, perisoxal, phenacetin, phenadoxone, phenazocine, phenazopyridine hydrochloride, phenocoll, phenoperidine, phenopyrazone, phenyl acetylsalicylate, phenylbutazone, phenyl salicylate, phenyramidol, piketoprofen, piminodine, pipebuzone, piperylone, piprofen, pirazolac, piritramide, piroxicam, pranoprofen, proglumetacin, proheptazine, promedol, propacetamol, propiram, propoxyphene, propyphenazone, proquazone, protizinic acid, ramifenazone, remifentanil, rimazolium metilsulfate, salacetamide, salicin, salicylamide, salicylamide o-acetic acid, salicylsulfuric acid, salsalte, salverine, simetride, sodium salicylate, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tolfenamic acid, tolmetin, tramadol, tropesin, viminol, xenbucin, ximoprofen, zaltoprofen and zomepirac (see The Merck Index, 12th Edition (1996), Therapeutic Category and Biological Activity Index, lists therein headed "Analgesic", "Anti-inflammatory" and "Antipyretic").

Particularly preferred combination therapies comprise use of a composition of the invention, for example a celecoxib or valdecoxib composition of the invention, with an opioid compound, more particularly where the opioid compound is codeine, meperidine, morphine or a derivative thereof.

The compound to be administered in combination with celecoxib can be formulated separately from the celecoxib or co-formulated with the celecoxib in a composition of the invention. Where celecoxib is co-formulated with a second drug, for example an opioid drug, the second drug can be formulated in immediate-release, rapid-onset, sustained-release or dual-release form.

In an embodiment of the invention, particularly where the cyclooxygenase-2 mediated condition is headache or migraine, the present selective cyclooxygenase-2 inhibitory drug composition is administered in combination therapy with a vasomodulator, preferably a xanthine derivative having vasomodulatory effect, more preferably an alkylxanthine compound.

Combination therapies wherein an alkylxanthine compound is co-administered with a selective cyclooxygenase-2 inhibitory drug composition as provided herein are embraced by the present embodiment of the invention whether or not the alkylxanthine is a vasomodulator and whether or not the therapeutic effectiveness of the combination is to any degree attributable to a vasomodulatory effect. The term "alkylxanthine" herein embraces xanthine derivatives having one or more C₁₋₄ alkyl, preferably methyl, substituents, and pharmaceutically acceptable salts of such xanthine derivatives. Dimethylxanthines and trimethylxanthines, including caffeine, theobromine and theophylline, are especially preferred. Most preferably, the alkylxanthine compound is caffeine.

The total and relative dosage amounts of the selective cyclooxygenase-2 inhibitory drug and of the vasomodulator or alkylxanthine are selected to be therapeutically and/or prophylactically effective for relief of pain associated with the headache or migraine. Suitable dosage amounts will depend on the particular selective cyclooxygenase-2 inhibitory drug and the particular vasomodulator or alkylxanthine selected. For example, in a combination therapy with celecoxib and caffeine, typically the celecoxib will be administered in a daily dosage amount of 50 mg to 1000 mg, preferably 100 mg to 600 mg, and the caffeine in a daily dosage amount of 1 mg to 500 mg, preferably 10 mg to 400 mg, more preferably 20 mg to 300 mg.

The vasomodulator or alkylxanthine component of the combination therapy can be administered in any suitable dosage form by any suitable route, preferably orally. The vasomodulator or alkylxanthine can optionally be coformulated with the selective cyclooxygenase-2 inhibitory drug in a single oral dosage form. Thus an oral fast-melt composition of the invention optionally comprises both an aminosulfonyl-comprising selective cyclooxygenase-2 inhibitory drug and a vasomodulator or alkylxanthine such as caffeine, in total and relative amounts consistent with the dosage amounts set out hereinabove.

The phrase "in total and relative amounts effective to relieve pain", with respect to amounts of a selective cyclooxygenase-2 inhibitory drug and a vasomodulator or alkylxanthine in a composition of the present embodiment, means that these amounts are such that (a) together these components are effective to relieve pain, and (b) each component is or would be capable of contribution to a pain-relieving effect if the other component is or were not present in so great an amount as to obviate such contribution.

### Ingredients of compositions of the invention

A composition of the invention comprises as active ingredient a selective cyclooxygenase-2 inhibitory drug as hereinabove described, and various pharmaceutically acceptable excipients. Excipients that must be present are a saccharide having low moldability as herein defined, a saccharide having high moldability as herein defined, and, in a presently preferred embodiment, a wetting agent. Optionally, a composition of the invention can contain one or more additional pharmaceutically acceptable excipients including, but not limited to, water-soluble lubricants, water-insoluble lubricants, disintegrants, glidants, sweeteners, flavoring agents, colorants, *etc*. Such optional additional components should be physically and chemically compatible with the other ingredients of the composition and must not be deleterious to the recipient.

### Active ingredient

The selective cyclooxygenase-2 inhibitory drug is present in an amount of 15% to 75%, preferably 15% to 60%, for example about 50%, by weight of the composition.

Particularly where the drug is one having a relatively high dosage requirement, such as celecoxib, a preferred composition comprises the drug in an amount of 15% to 75%, preferably 30% to 75%, and more preferably 45% to 75%, for example about 60%, by weight of the composition. We have been surprised that through the process of the present invention we have been able to formulate such an extremely hydrophobic drug as celecoxib at such high concentration in an oral fast-melt tablet.

### Low moldability saccharide

Presently preferred low moldability saccharides include lactose and mannitol, particularly mannitol in its non-direct compression or powder form as described in Handbook of Pharmaceutical Excipients, 3rd Ed. (2000), Pharmaceutical Press, pp. 324-328. One or more low moldability saccharides are present in compositions of the invention in a total amount of 10% to 90%, preferably 15% to 60%, and more preferably 25% to 50%, for example about 40%, by weight of the composition.

### High moldability saccharide

Presently preferred high moldability saccharides include maltose, maltitol and sorbitol. Alternatively, certain oligosaccharides can be useful. The oligosaccharide used is not particularly limited so long as it shows rapid dissolution in the oral cavity and consists of two or more monosaccharide residues. Where an oligosaccharide is used, one consisting of 2 to 6 monosaccharide residues is preferable, and the type and combination of monosaccharide residues constituting the oligosaccharide are not limited. Particularly preferred high moldability saccharides are maltose and maltitol, more particularly maltose.

One or more high moldability saccharides are present in a total amount of 1% to 10%, preferably 1% to 7.5%, and more preferably 1% to 5%, by weight of the composition.

The weight ratio of high moldability saccharide to low moldability saccharide in a fast-melt tablet of the invention is important in maintaining a combination of acceptable tablet hardness and rapid intraoral disintegration. A suitable ratio is 2 to 20 parts by weight, preferably 5 to 10 parts by weight, and more preferably 5 to 7.5 parts by weight, of the high moldability saccharide per 100 parts by weight of the low moldability saccharide.

If the ratio of high to low moldability saccharide is less than about 2:100 by weight, tablets typically do not achieve their desired hardness, resulting in increased breakage during storage, transportation or handling. Alternatively, if the ratio of high to low moldability saccharide exceeds about 20:100 by weight, the tablets become too hard and desired rapid disintegration in the oral cavity is not achieved.

### Wetting agents

In a preferred embodiment, compositions of the present invention comprise one or more pharmaceutically acceptable wetting agents. Surfactants, hydrophilic polymers and certain clays can be useful as wetting agents to aid in wetting of a hydrophobic drug, such as celecoxib, by the granulation fluid during wet granulation. Where compositions of the present invention are made by the fluid bed granulation process, it is particularly advantageous that the composition contain a wetting agent. Importantly, however, where a relatively low dose cyclooxygenase-2 inhibitory drug such as valdecoxib is used and the concentration of the drug in the composition is therefore relatively low, a wetting agent may not be required, particularly if a glidant, for example silicon dioxide, is used.

Examples of surfactants that can be used as wetting agents in compositions of the present invention include quaternary ammonium compounds, for example benzalkonium chloride, benzethonium chloride and cerylpyridinium chloride, dioctyl sodium sulfosuccinate, polyoxyethylene alkylphenyl ethers, for example nonoxynol 9, nonoxynol 10, and octoxynol 9, poloxamers (polyoxyethylene and polyoxypropylene block copolymers), polyoxyethylene fatty acid glycerides and oils, for example polyoxyethylene (8) caprylic/capric mono- and diglycerides (*e*.*g*., Labrasol™ of Gattefossé), polyoxyethylene (35) castor oil and polyoxyethylene (40) hydrogenated castor oil; polyoxyethylene alkyl ethers, for example polyoxyethylene (20) cetostearyl ether, polyoxyethylene fatty acid esters, for example polyoxyethylene (40) stearate, polyoxyethylene sorbitan esters, for example polysorbate 20 and polysorbate 80 (*e*.*g*., Tween™ 80 of ICI), propylene glycol fatty acid esters, for example propylene glycol laurate (*e*.*g*., Lauroglycol™ of Gattefossé), sodium lauryl sulfate, fatty acids and salts thereof, for example oleic acid, sodium oleate and triethanolamine oleate, glyceryl fatty acid esters, for example glyceryl monostearate, sorbitan esters, for example sorbitan monolaurate, sorbitan monooleate, sorbitan monopalmitate and sorbitan monostearate, tyloxapol, and mixtures thereof. Sodium lauryl sulfate is a preferred wetting agent in compositions of the present invention.

One or more wetting agents, if desired, are present in compositions of the present invention in a total amount of 0.05% to 5%, preferably 0.075% to 2.5%, and more preferably 0.25% to 1 %, for example about 0.5%, by weight of the composition.

### Water-insoluble lubricants

Compositions of the present invention optionally comprise one or more pharmaceutically acceptable water-insoluble lubricants as a carrier material. Suitable water-insoluble lubricants include, either individually or in combination, glyceryl behapate (*e.g.* Compritol™ 888), stearates (magnesium, calcium, and sodium), stearic acid, hydrogenated vegetable oils (*e.g*., Sterotex™), colloidal silica, talc, waxes and mixtures thereof. Optionally a water-insoluble lubricant can be used in mixture with a wetting agent, as for example in calcium stearate/sodium lauryl sulfate mixtures (*e.g*., Sterowet™).

Magnesium stearate, stearic acid and mixtures thereof are preferred water-insoluble lubricants.

One or more water-insoluble lubricants optionally are present in compositions of the present invention in a total amount of 0.05% to 5%, preferably

0.75% to 2.5%, and more preferably 1% to 2%, for example, about 1.5%, by weight of the composition.

### Water-soluble lubricants

Compositions of the present invention optionally comprise one or more pharmaceutically acceptable water-soluble lubricants. Water-soluble lubricants can help to improve tablet dissolution characteristics. Water-soluble lubricants that can be used in compositions of the present invention either individually or in combination include, for example, boric acid, sodium benzoate, sodium acetate, sodium fumarate, sodium chloride, DL-leucine, polyethylene glycols (*e.g*., Carbowax™ 4000 and Carbowax™ 6000), and sodium oleate.

### Disintegrants

Compositions of the present invention optionally comprise one or more pharmaceutically acceptable disintegrants, particularly for tablet formulations. However, the oral fast-melt tablets provided herein typically disintegrate rapidly in the oral cavity and have no requirement for added disintegrant. Suitable disintegrants, if desired, include, either individually or in combination, starches, sodium starch glycolate, clays (such as Veegum™ HV), celluloses (such as purified cellulose, methylcellulose, sodium carboxymethylcellulose and carboxymethylcellulose), croscarmellose sodium, alginates, pregelatinized corn starches (such as National™ 1551 and National™ 1550), crospovidone, and gums (such as agar, guar, locust bean, karaya, pectin and tragacanth gums). Disintegrants can be added at any suitable step during the preparation of the composition, particularly prior to granulation or during a blending step prior to tablet compression. Croscarmellose sodium and sodium starch glycolate are preferred disintegrants.

One or more disintegrants optionally are present in a total amount of 0.5% to 7.5%, preferably 1 % to 5%, and more preferably 1 % to 3.5%, by weight of the composition.

Optionally, an effervescent salt can be used as a disintegrant and to enhance organoleptic properties of a fast-melt tablet of the invention.

### Glidants

Compositions of the present invention optionally comprise one or more pharmaceutically acceptable glidants, for example to enhance flow of tableting material into tablet dies, to prevent sticking of tableting material to punches and dies, or to produce tablets having a sheen. Glidants may be added at any suitable step during preparation of the composition, particularly prior to granulation or during a blending step prior to tablet compression.

Without being bound by theory, it is believed that, in some situations, glidants, for example talc or silicon dioxide, act to reduce interfacial tension between drug particles, having the effect of inhibiting and/or reducing drug agglomeration, act to decrease electrostatic charges on the surface of drug powders, and act to reduce interparticular friction and surface rugosity of drug particles. See, for example, York (1975) J. Pharm. Sci., 64(7), 1216-1221. Use of a glidant such as silicon dioxide, therefore, can eliminate or reduce the need for a wetting agent in certain instances, for example, when formulating low dose selective cyclooxygenase-2 inhibitory drugs such as valdecoxib.

Silicon dioxide is a preferred glidant. Suitable silicon dioxide products for use in preparing compositions of the invention include fumed silica or colloidal silica (*e*.*g*., Cab-O-Sil™ of Cabot Corp. and Aerosil™ of Degussa). Silicon dioxide, when present in compositions of the invention, is present in a total amount of 0.05% to 5%, preferably 0.1% to 2%, and more preferably 0.25% to 1%, for example, about 0.5%, by weight of the composition.

### Sweetening agents

Compositions of the present invention optionally comprise one or more pharmaceutically acceptable sweeteners. Non-limiting examples of sweeteners that can be used in compositions of the present invention include mannitol, propylene glycol, sodium saccharin, acesulfame K, neotame, aspartame, *etc.*

### Flavoring agents

Compositions of the present invention optionally comprise one or more pharmaceutically acceptable flavoring agents. Non-limiting examples of flavoring agents that can be used in compositions of the present invention include peppermint, spearmint, grape, cherry, strawberry, lemon, *etc.*

### Tablet characteristics

### Size and shape

In a preferred embodiment, compositions of the invention are in the form of discrete solid dosage units, most preferably tablets. Tablets of the invention can be made to any desired size, for example 8 mm, 10 mm, 12 mm, *etc*.; shape, for example round, oval, oblong, *etc*.; weight; and thickness. Optionally, solid dosage units of the invention may have etchings or monograms on one or both sides.

### Disintegration

**Tablet** compositions of the invention disintegrate within 30 to 200 seconds, and still more preferably within 30 to 150 seconds, in a standard *in vitro* disintegration assay (conducted according to U.S. Pharmacopeia 24 (2000), Test No. 701).

Additionally, preferred tablet compositions of the invention disintegrate within 5 to 60 seconds, more preferably within 5 to

40 seconds, and still more preferably within 5 to 30 seconds, for example about 25 seconds, after placement in the oral cavity of a subject.

### Hardness

Solid dosage fonns of the invention have a hardness that can depend on size and shape as well as on composition, among other characteristics. Tablet hardness can be measured by any method known in the art, for example by a tablet hardness meter (*e.g*., Schleuniger). Preferably, compositions of the invention have a hardness of 9.8 to 98 N (1 to 10 kp), and more preferably of 9.8 to 59 N (1 to 6 kp).

In a presently preferred embodiment, solid dosage forms of the invention have sufficient hardness for handling and, therefore, can be put into practical use in the same manner as the case of ordinary tablets. The term "sufficient hardness for handling" as used herein means a hardness which can withstand removal from at least a standard type of blister packaging, or such a hardness as will withstand other handling such as packaging, delivery, carrying and the like.

Tablets of the invention preferably have a minimum hardness so as to resist breakage of the tablet during removal from standard blister packaging by pushing the tablet through a cover sheet. A suitable hardness is 9.8 N (1 kp) or more for a tablet having a diameter of about 8 mm, 14.7 N (1.5 kp) or more for a tablet having a diameter of about 10 mm, and 19.6 N (2 kp) or more when the tablet has a diameter of about 12 mm.

In another presently preferred embodiment, tablets of the invention have sufficient hardness such that a plurality of such tablets can be packaged together, for example in a glass or plastic bottle, without individual packaging, yet do not exhibit substantial breakage or sticking and/or melding together during normal shipping and handling. Tablets intended for such packaging preferably have a hardness of 29.4 N (3 kp) or more.

### Packaging

Compositions of the invention can be packaged in any suitable manner known in the art. For example, a multiplicity of fast-melt tablets can be packaged together, for example in a glass or plastic bottle or container. Alternatively, fast-melt tablets of the invention can be individually wrapped, for example in plastic or foil, or packaged in known forms of blister packaging. Blister packaging with improved force distribution properties such as is disclosed - U.S. Patent No. 5,954,204 to Grabowski can be especially useful to package fast-melt tablets of the invention.

### Administration of fast-melt tablets

Compositions of the present invention can be taken by a subject by any oral administration means in accordance with the subject's choice or condition. For example, fast-melt tablets of the invention can be taken without water. Upon placement in the oral cavity and especially in the cheek or above the tongue, such a tablet is exposed to saliva and rapidly disintegrates and dissolves therein. The rate of disintegration and/or dissolution increases further when an intraoral pressure, for example a pressure between the palate and tongue or a licking or sucking pressure, is applied to the tablet.

Alternatively, a tablet of the present invention can be taken with the aid of water in an amount sufficient to wet the oral cavity and to assist in disintegration of the tablet. Also, a tablet of the invention can be swallowed together with a small amount of water after complete or partial disintegration in the oral cavity. Compositions of the invention can also be swallowed directly with water.

### Method to make fast-melt tablets

The process described below is a non-limiting, illustrative method to make celecoxib fast-melt tablets. Importantly, specific settings and parameters of the production process can be readily optimized by one of skill in the art in order to produce tablets with particularly desired characteristics.

In this illustrative process, celecoxib and low moldability mannitol are de-lumped in a mill or grinder and blended to form a drug powder mixture. Next, this drug powder mixture is wet granulated, preferably by fluid bed granulation, with sodium lauryl sulfate and maltose solutions to form granules. If the granules are not dried during granulation, for example as is the case in fluid bed granulation, they are dried after granulation, for example in an oven. The resulting dried granules are then milled to form a milled granulate. The milled granulate is then optionally blended with flavor, sweetener and lubricants in a tumble blender to form a tablet blend. The resulting tablet blend is then compressed on a rotary tablet press to a target tablet weight and hardness. The resulting tablets are then subjected to treatment, for example air flow treatment, in a humidity-controlled chamber with the effect of increasing tablet hardness.

### Wet granulation

Fluid bed granulation is the preferred method of wet granulation in processes of the invention, although any known wet granulation method, for example high-shear granulation or pan granulation, can be used.

Illustratively, in fluid bed granulation, celecoxib, low moldability mannitol, and any other desired excipients are mixed together and sized in a mill or grinder. Next, the resulting drug powder mixture is granulated in a fluid bed by spraying a liquid solution of a high moldability saccharide and a wetting agent onto the mixture. The wet granules are then fluid bed dried.

After fluid bed granulation is complete, the resulting dried granules are then blended with any further desired excipients and then compressed into tablets.

Alternatively, in high-shear wet granulation, celecoxib, mannitol and any other desired excipients are blended under high shear in a granulator. Next, a liquid solution of high moldability saccharide and wetting agent are added to the resulting drug powder mixture under continuing high shear, thereby forming wet granules.

After high-shear granulation is complete, the resulting granules are then dried, for example, in an oven, microwave or fluid bed. The dried granules are then transferred to a blender for addition of any other desired excipients to form a tablet blend, which is then compressed.

Whether fluid bed or high-shear granulation is used, the celecoxib, low moldability mannitol and other excipients can, in an alternative process, be separately granulated and the resulting granules mixed together prior to compression.

### Tablet compression

Compression is the process by which an appropriate volume of a tablet blend of granules produced as described above is compressed between an upper and lower punch to consolidate material into a single solid dosage form such as a tablet. In processes for manufacture of fast-melt tablets of the present invention, any suitable means for compression can be used including, for example, a single punch tablet machine or a high speed rotary tablet press. The tableting pressure is not limited, and an appropri ate pressure can be selected depending on the desired hardness and dissolution properties of the resulting tablets. Where tablets are to undergo temperature and humidity treatment as described immediately below, the tablets are preferably compressed to an initial hardness (prior to temperature and humidity treatment) of 7.3 to 14.7 N (0.75 to 1.5 kp).

### Temperature and humidity treatment

Optionally, tablets of the invention can undergo heat and humidity treatment after the tablet compression step. Such treatment can be performed in a humidity chamber, for example, to increase hardness of the tablets. Illustratively, during this treatment, tablets are first subjected to low temperature, high humidity air flow conditions, for example, 25°C to 32°C and about 80% relative humidity, for a period of 45 to 120 minutes. Tablets are then subjected to high temperature, low humidity conditions, for example 35°C to 50°C and 30% relative humidity for a period.of 45 to 120 minutes. Without being bound by theory, it is believed that treatment of fast-melt tablets in a low temperature/high humidity chamber followed by treatment in a high temperature/low humidity chamber increases tablet hardness and reduces tablet friability without sacrificing desired fast-melt characteristics such as rapid disintegration and rapid dissolution.

### EXAMPLES

The following examples illustrate aspects of the present invention.

### Example 1

Celecoxib fast-melt tablet formulations F1 to F7 were prepared having components as shown in Table 1, below. The formulations were prepared according to the following method.
1. Celecoxib and low moldability mannitol were de-lumped in a Co-mil producing a drug powder mixture.
2. The drug powder mixture was charged to a Glatt fluid bed processor and pre-heated. Inlet air was used to provide fluidization of the powder mixture. An aqueous sodium lauryl sulfate (1% by weight) solution and an aqueous maltose solution (5% to 6.5% by weight) were sprayed onto the fluidized powder bed resulting in wet granules. The wet granules were then fluid bed dried.
3. The resulting dried granules were subjected to a milling step through a Co-mil to form a milled granulate.
4. The milled granulate was blended with flavoring agent (spearmint flavor), sweetening agent (acesulfame K) and lubricants (magnesium stearate and stearic acid) in a tumble blender for approximately 5 to 10 minutes to form a blend.
5. The blend was then compressed on a rotary tablet press using a tooling size of 11.1 or 12.7 mm to form tablets having an initial hardness of 4.9 to 14.7 N (0.5 to 1.5 kp), and a target weight corresponding to a 200 mg dose of celecoxib.
6. The tablets were subjected to treatment in a chamber through which air at two specified sets of temperatures and relative humidity conditions was circulated. First, air at a temperature of 25°C and a relative humidity of 80% was circulated through the chamber for about 45 minutes. Second, air at a temperature of 50°C and a relative humidity of 30% was circulated through the chamber for about 45 minutes. Target final hardness was 29 to 39 N (3 to 4 kp).

**Table 1. Composition (%) of fast-melt tablets F1 to F7**

| Formulation No. | **F1** | **F2** | **F3** | **F4** | **F5** | **F6** | **F7** |
|---|---|---|---|---|---|---|---|
| Tooling (mm) | 11.1 | 11.1 | 12.7 | 12.7 | 11.1 | 11.1 | 11.1 |
| Celecoxib | 25.0 | 33.0 | 50.0 | 50.0 | 60.0 | 50.0 | 50.0 |
| Mannitol ¹ | 66.75 | 58.75 | 40.25 | 41.75 | 31.75 | 41.75 | 39.25 |
| Maltose | 5.0 | 5.0 | 6.5 | 5.0 | 5.0 | 5.0 | 7.5 |
| Magnesium stearate | 0.75 | 0.75 | 0.75 | 0.75 | 0.75 | 0.75 | 1.0 |
| Stearic acid | 0.75 | 0.75 | 0.75 | 0.75 | 0.75 | 0.75 | 1.0 |
| Sodium lauryl sulfate | 1.0 | 1.0 | 1.0 | 1.0 | 1.0 | 3.0 | 1.0 |
| Acesulfame K | 0.5 | 0.5 | 0.5 | 0.5 | 0.5 | 4.0 | 0.5 |
| Spearmint flavor | 0.25 | 0.25 | 0.25 | 0.25 | 0.25 | 0.25 | 0.25 |
| Total (%) | 100 | 100 | 100 | 100 | 100 | 100 | 100 |
| Final Weight (mg) | 400 | 400 | 400 | 400 | 400 | 400 | 400 |

| | | | | | | | |
|---|---|---|---|---|---|---|---|
| ¹ low moldability mannitol | | | | | | | |

Tablet hardness was determined by measuring the degree of force required to break the tablets. Tablet hardness data for formulations F1 to F7 are provided below in Table 2.

Disintegration profiles of tablets were evaluated in a standard in *vitro* disintegration assay (U.S. Pharmacopeia 24 (2000), Test No. 701) under the following conditions. Tablets were placed into a basket-rack assembly containing 6 open-ended glass tubes held vertically upon a 10-mesh stainless steel wire screen. The baskets were then raised and lowered in an immersion fluid consisting of water at 37°C. at a frequency of 29 to 32 cycles per minute. Complete disintegration of tablets was recorded when no residue of the tablet.remained on the screen of the test apparatus except that consisting of a soft mass having no palpably firm core. Tablet *in vitro* disintegration data for formulations F1 to F5 are provided below in Table 2, below.

*In vivo* disintegration profiles of tablets were evaluated in human subjects according to the following procedure. A subject placed a fast-melt tablet on his or her tongue and immediately started a chronometer. The subject gently moved the tablet against the upper part of the mouth with the tongue, creating gentle tumble action on the tablet without biting on it. The subject then stopped the chronometer immediately after the last noticeable granule disintegrated. Time to disintegration was then recorded.

*In vivo* disintegration data for formulations F1 to F7 are provided below in Table 2, below.

**Table 2. Properties of celecoxib fast-melt tablet formulations F1 to F7**

| Formulation No. | **F1** | **F2** | **F3** | **F4** | **F5** | **F6** | **F7** |
|---|---|---|---|---|---|---|---|
| Mean *in vitro* disintegration time (sec.) | 46 | 99.5 | 99.3 | 97.4 | 63.0 | - | - |
| Mean *in vivo* disintegration time (sec.) | 24.0 | 27.5 | 32.0 | 31.0 | 30.0 | 30.8 | 30.7 |
| Mean hardness (kp)* | 3.6 | 3.6 | 2.4 | 4.0 | 2.7 | 3.5 | 3.2 |

| | | | | | | | |
|---|---|---|---|---|---|---|---|
| * For conversion to N multiply by 9.8067. | | | | | | | |

### Example 2

Celecoxib fast-melt tablet formulations F8 and F9 were prepared having components as shown in Table 3, below. The formulations were prepared according to the following method.
1. Celecoxib, silicon dioxide and low moldability mannitol were de-lumped in a Co-mil producing a drug powder mixture.
2. The drug powder mixture was charged to a Glatt fluid bed processor and pre-heated. Inlet air was used to provide fluidization of the powder bed, and an aqueous sodium lauryl sulfate solution (2.5% by weight) and an aqueous maltose solution (15% by weight) were sprayed onto the fluidized powder bed resulting in wet granules. Importantly, no material stuck to the walls of the processor. The wet granules were then fluid bed dried.
3. The resulting dried granules were subjected to a milling step through a Co-mil to form a milled granulate.
4. The milled granulate was blended with flavoring agent (acesulfame K and peppermint flavor) and lubricants (magnesium stearate and stearic acid) in a tumble blender for about 5 minutes to form a blend.
5. The blend was then compressed in a Korsch press using a tooling of 11.9 mm, to form tablets having an initial target hardness of about 9.81 N (1.0 kp) and a final tablet target weight of 400 mg.
6. The tablets were subjected to treatment in a chamber through which air at two specified sets of temperatures and relative humidity conditions was circulated. First, air at a temperature of 25°C and a relative humidity of 80% was circulated through the chamber for about 40 minutes. Second, air at a temperature of 50°C and relative humidity of 30% was circulated through the chamber for about 60 minutes.

**Table 3. Composition (mg) of celecoxib fast-melt formulations F8 and F9**

| Formulation No. | **F8** | **F9** |
|---|---|---|
| Tooling (mm) | 11.9 | 12 |
| Celecoxib | 200 | 200 |
| Mannitol¹ | 165 | 167 |
| Maltose | 20.0 | 20.0 |
| Magnesium stearate | 3.0 | 3.0 |
| Silicon dioxide | 2.0 | 2.0 |
| Stearic acid | 3.0 | 3.0 |
| Sodium lauryl sulfate | 4.0 | 2.0 |
| Acesulfame K | 2.0 | 2.0 |
| Spearmint flavor | 1.0 | 1.0 |
| Total | 400 | 400 |

| | | |
|---|---|---|
| ¹ low moldability mannitol | | |

**Table 4. Properties of celecoxib fast-melt tablet formulations F8 and F9**

| Formulation No. | **F8** | **F9** |
|---|---|---|
| Mean *in vitro* disintegration time (min.) | 1.01 | 2.13 |
| Mean *in vivo* disintegration time (sec.) | 23 | - |
| Final hardness (kp)* | 3.2-4.0 | 4.6-5.4 |

| | | |
|---|---|---|
| * For conversion to N multiply by 9.8067. | | |

### Example 3

Valdecoxib fast-melt tablet formulations F10 and F11 were prepared having components as shown in Table 4, below. The formulations were prepared according to the following method.
1. Valdecoxib, silicon dioxide and low moldability mannitol were de-lumped in a Co-mil producing a drug powder mixture.
2. The drug powder mixture was charged to a Glatt fluid bed processor and pre-heated. Inlet air was used to provide fluidization of the powder bed, and an aqueous maltose solution (15% by weight) was sprayed onto the fluidized powder bed resulting in wet granules. Importantly, no material stuck to the walls of the processor. The wet granules were then fluid bed dried.
3. The resulting dried granules were subjected to a milling step through a Co-mil to form a milled granulate.
4. The milled granulate was blended with flavoring agent (acesulfame K and peppermint or spearmint flavor) and lubricants (magnesium stearate and stearic acid) in a tumble blender for about 5 minutes to form a blend.
5. The blend was then compressed in a Korsch press using a tooling of 11.9 mm, to form tablets having an initial target hardness of about 9.81 N (1.0 kp) and a final tablet target weight of 400 mg.
6. The tablets were subjected to treatment in a chamber through which air at two specified sets of temperatures and relative humidity conditions was circulated. First, air at a temperature of 25°C and a relative humidity of 80% was circulated through the chamber for about 40 minutes. Second, air at a temperature of 50°C and a relative humidity of 30% was circulated through the chamber for about 60 minutes.

**Table 4. Composition (mg) of valdecoxib fast-melt formulations F10 and F11**

| Formulation No. | **F10** | **F11** |
|---|---|---|
| Tooling (mm) | 11.9 | 11.9 |
| Valdecoxib | 40 | 40 |
| Mannitol¹ | 326 | 326 |
| Maltose | 20.0 | 20.0 |
| Magnesium stearate | 2.0 | 2.0 |
| Silicon dioxide | 2.0 | 2.0 |
| Stearic acid | 6.0 | 6.0 |
| Acesulfame K | 2.0 | 2.0 |
| Spearmint flavor | 2.0 | - |
| Peppermint flavor | - | 2.0 |
| Total | 400 | 400 |

| | | |
|---|---|---|
| ¹ low moldability mannitol | | |

## Claims

1. A process for preparing an oral fast-melt pharmaceutical composition, the process comprising
(a) a step of wet granulating a selective cyclooxygenase-2 inhibitory drug together with a liquid binding agent comprising a saccharide having high moldability, and
(b) a step of blending with the drug a saccharide having low moldability, wherein said steps (a) and (b) occur in any order or simultaneously to result in formation of granules;
wherein the process incorporates means to inhibit agglomeration of the drug, said means selected from the group consisting of addition of a wetting agent, means for pre-wetting the material to be granulated, and means for increasing air flow along the periphery of the granulation bowl; wherein said wetting agent is selected from the group consisting of surfactants, hydrophilic polymers and clays; wherein said surfactant is selected from the group consisting of quaternary ammonium compounds, dioctyl sodium sulfosuccinate, polyoxyethylene alkylphenyl ethers, polyoxyethylene block copolymers, polyoxypropylene block copolymers, polyoxyethylene fatty acid glycerides, polyoxyethylene fatty acid oils, polyoxyethylene alkyl ethers, polyoxyethylene fatty acid esters, polyoxyethylene sorbitan esters, propylene glycol fatty acid esters, sodium lauryl sulfate, fatty acids, salts of fatty acids, glyceryl fatty acid esters, sorbitan esters, tyloxapol, and mixtures thereof;
wherein the selective cyclooxygenase-2 inhibitory drug is a compound having the formula: 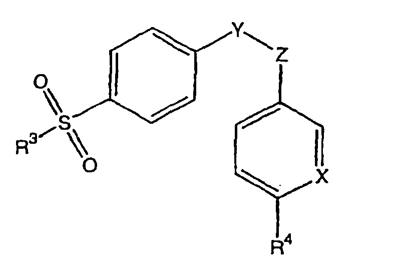 where R³ is a methyl or amino group, R⁴ is hydrogen or a C₁₋₄ alkyl or alkoxy group, X is N or CR⁵ where R⁵ is hydrogen or halogen, and Y and Z are independently carbon or nitrogen atoms defining adjacent atoms of a five- to six-membered ring that is unsubstituted or substituted at one or more positions with oxo, halo, methyl or halomethyl groups;
wherein said selective cyclooxygenase-2 inhibitory drug is present in an amount of 15% to 75% by weight of the composition;
wherein the saccharide having high moldability shows a hardness of 19.6 N (2 kp) or more when 150 mg of the saccharide is made into a tablet using a punch of 8 mm in diameter under a pressure of 10 to 50 kg/cm²;
wherein the saccharide having low moldability shows a hardness of less than 19.6 N (2 kp) when 150 mg of the saccharide is made into a tablet using a punch of 8 mm in diameter under a pressure of 10 to 50 kg/cm²; and
wherein the fast-melt pharmaceutical composition disintegrates within 30 to 200 seconds in a standard in vitro disintegration assay conducted according to U.S. Pharmacopeia 24 (2000), Test No. 701.

2. The process of Claim 1 wherein said step (b) occurs prior to or simultaneously with said step (a).

3. The process of Claim 1 wherein said wet granulating step comprises fluid bed granulation.

4. The process of Claim 1 wherein the five- to six-membered ring is selected from cyclopentenone, furanone, methylpyrazole, isoxazole and pyridine rings substituted at no more than one position.

5. The process of Claim 1 wherein the selective cyclooxygenase-2 inhibitory drug is selected from celecoxib, deracoxib, valdecoxib, rofecoxib, etoricoxib, 2-(3,5-difluorophenyl)-3-[4-(methylsulfonyl)phenyl]-2-cyclopenten-1-one, (S)-6,8-dichloro-2-(trifluoromethyl)-2H-1-benzopyran-3-carboxylic acid and 2-(3,4-difluorophenyl)-4-(3-hydroxy-3-methyl-1-butoxy)-5-[4-(methylsulfonyl)phenyl]-3-(2H)-pyridazinone.

6. The process of Claim 1 wherein the selective cyclooxygenase-2 inhibitory drug is celecoxib.

7. The process of Claim 1 wherein the selective cyclooxygenase-2 inhibitory drug is valdecoxib.

8. The process of Claim 1 wherein said saccharide having low moldability is selected from lactose, mannitol, glucose, sucrose and xylitol.

9. The process of Claim 1 wherein said saccharide having low moldability is mannitol of powder grade.

10. The process of Claim 1 wherein said saccharide having high moldability is selected from maltose, maltitol, sorbitol and oligosaccharides having 2 to 6 monosaccharide residues.

11. The process of Claim 1 wherein said saccharide having high moldability is maltose.

12. The process of Claim 1 wherein said wetting agent is added in a total amount of 0.05% to 5% by weight of the composition.

13. The process of Claim 1 further comprising addition of a glidant.

14. The process of claim 13 wherein said glidant is silicon dioxide.

15. The process of Claim 13 wherein said glidant is added in a total amount of 0.05% to 5% by weight of the composition.

16. The process of Claim 1 wherein said selective cyclooxygenase-2 inhibitory drug is present in a total amount of 45% to 75% by weight of the composition.

17. The process of Claim 1 wherein said saccharide having high moldability is present in a total amount of 1% to 10% by weight of the composition.

18. The process of Claim 1 wherein said saccharide having low moldability is present in a total amount of 10% to 90% by weight of the composition.

19. The process of Claim 1 wherein the weight ratio of said saccharide having high moldability to said saccharide having low moldability is 2:100 to 20:100.

20. The process of Claim 1, further comprising
(c) a step of blending said granules with at least one of a lubricant, a sweetening agent and a flavoring agent to form a tableting blend, and
(d) a step of compressing the tableting blend to form oral fast-melt tablets.

21. The process of Claim 20 wherein parameters are set in said compressing step (d) to provide tablets having a hardness of 9.8 to 98 N (1 to 10 kp).

22. The process of claim 1 wherein step (b) is a step of blending with the drug a saccharide having low moldability and a glidant.

23. An oral fast-melt pharmaceutical composition prepared by the process of any of Claims 1 through 22.

24. An oral fast-melt composition of Claim 23 comprising a selective cyclooxygenase-2 inhibitory drug dispersed in a matrix comprising a saccharide of low moldability and a saccharide of high moldability.

25. The composition of Claim 24 further comprising a wetting agent.

26. The composition of Claim 25 wherein said wetting agent is defined as in claim 1.

27. The composition of Claim 24 further comprising a glidant.

28. The composition of Claim 27 wherein said glidant is as defined in claim 14 or 15.

29. The composition of Claim 24 wherein the selective cyclooxygenase-2 inhibitory drug is present in an amount of 1% to 75% by weight of the composition.

30. The composition of Claim 24 wherein the selective cyclooxygenase-2 inhibitory drug is present in an amount of 45% to 75% by weight of the composition.

31. The composition of Claim 24 wherein said saccharide having low moldability is as defined in claims 8 and 9.

32. The composition of Claim 24 wherein said saccharide having low moldability is present in an amount of 10% to 90% by weight of the composition.

33. The composition of Claim 24 wherein said saccharide having high moldability is as defined in claims 10 and 11.

34. The composition of Claim 24 wherein said saccharide having high moldability is present in an amount of 1% to 10% by weight of the composition.

35. The composition of Claim 24 wherein the weight ratio of said saccharide having high moldability to said saccharide having low moldability is 2:100 to 20:100.

36. The composition of Claim 24 that is a tablet.

37. The tablet of Claim 36 that disintegrates within 30 to 300 seconds in a standard *in vitro* disintegration assay.

38. The composition of Claim 36 that disintegrates within 5 to 60 seconds after placement in the oral cavity of a subject.

39. Use of a composition of claims 24 to 38 for the preparation of a medicament for the oral treatment of a medical condition or disorder in a mammalian subject selected from the group consisting of inflammation, pain, fever, arthritis, arthritic disorders, asthma, bronchitis, menstrual cramps, preterm labor, tendinitis, bursitis, allergic neuritis, cytomegalovirus infectivity, apoptosis including HIV-induced apoptosis, lumbago, liver disease, psoriasis, eczema, acne, burns, dermatitis and ultraviolet radiation damage, post-operative inflammation, gastrointestinal conditions, migraine headaches, periarteritis nodosa, thyroiditis, aplastic anemia, Hodgkin's disease, sclerodoma, rheumatic fever, type I diabetis, neuromuscular junction disease, white matter disease, sarcoidosis, nephrotic syndrome, Behcet's syndrome, polymyositis, gingivitis, nephritis, hypersensitivity, swelling occurring after injury, myocardial ischemia, ophthalmic diseases, acute injury to eye tissue, pulmonary inflammation, osteoporosis, central nervous system disorders, allergic rhinitis, respiratory distress syndrome, endotoxin shock syndrome, inflammation-related cardiovascular disorders, vascular diseases, coronary artery disease, aneurysm, vascular rejection, arteriosclerosis, atherosclerosis, myocardial infarction, embolism, stroke, thrombosis, angina, coronary plaque inflammation, angiogenesis-related disorders, neoplasia, corneal graft rejection, ocular neovascularization, retinal neovascularization, macular degeneration, retrolental fibroplasia, glaucoma, gastric ulcer, hemangiomas, disorders of the female reproductive system, fibrosis that occurs with radiation therapy and adenomatous polyps.

40. The use of Claim 39 wherein said mammalian subject is a human subject.

41. The use of Claim 40 that further comprises combination therapy with one or more drugs selected from opioids and other analgesics.

42. The use of Claim 40 that further comprises combination therapy with an opioid compound selected from codeine, meperidine and morphine.

## Patentansprüche

1. Verfahren zum Herstellen einer oralen schnell zergehenden pharmazeutischen Zusammensetzung, wobei das Verfahren umfasst:
(a) eine Stufe des Feuchtgranulierens eines selektiven Cyclooxygenase-2-inhibierenden Wirkstoffs, zusammen mit einem Flüssigkeits-bindenden Mittel, umfassend ein Saccharid mit hoher Formbarkeit, und
(b) eine Stufe des Mischens eines Saccharids mit hoher Formbarkeit mit dem Wirkstoff, wobei die Stufen (a) und (b) in einer beliebigen Reihenfolge oder simultan erfolgen, um in der Bildung von Granulaten zu resultieren;
wobei das Verfahren Mittel einschließt, um die Agglomeration des Wirkstoffs zu inhibieren, wobei die Mittel ausgewählt sind aus der Gruppe, bestehend aus Zugabe eines Benetzungsmittels, Mitteln zum Vorbenetzen des Materials, das granuliert werden soll, und Mitteln zum Erhöhen des Luftflusses entlang des Umfangs des Granulierungsbeckens; wobei das Benetzungsmittel ausgewählt ist aus der Gruppe, bestehend aus oberflächenaktiven Mitteln, hydrophilen Polymeren und Tonen; wobei das oberflächenaktive Mittel ausgewählt ist aus der Gruppe, bestehend aus quaternären Ammoniumverbindungen, Dioctylnatriumsulfosuccinat, Polyoxyethylenalkylphenylethern, Polyoxyethylen-Block-Copolymeren, Polyoxypropylen-Block-Copolymeren, Polyoxyethylenfettsäureglyceriden, Polyoxyethylenfettsäureölen, Polyoxyethylenalkylethern, Polyoxyethylenfettsäureestern, Polyoxyethylensorbitanestern, Propylenglycolfettsäureestern, Natriumlaurylsulfat, Fettsäuren, Salzen von Fettsäuren, Glycerylfettsäureestern, Sorbitanestern, Tyloxapol und Gemischen davon;
wobei der selektive Cyclooxygenase-2-inhibierende Wirkstoff eine Verbindung mit der Formel 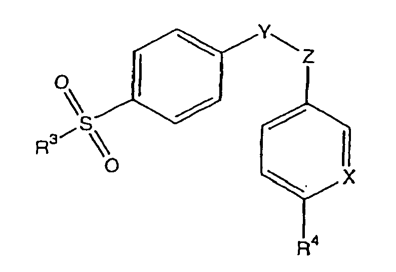 ist, worin R³ eine Methyl- oder Aminogruppe ist, R⁴ Wasserstoff oder eine C₁₋₄-Alkylgruppe oder Alkoxygruppe ist, X N oder CR⁵ ist, wobei R⁵ Wasserstoff oder Halogen ist und Y und Z unabhängig Kohlenstoff- oder Stickstoffatome sind, die benachbarte Atome eines fünf- oder sechsgliedrigen Ringes definieren, der an einer oder mehreren Stellen unsubstituiert oder substituiert ist mit Oxo-, Halogen-, Methyl- oder Halogenmethylgruppen;
wobei der selektive Cyclooxygenase-2-inhibierende Wirkstoff in einer Menge von 15 Gew.-% bis 75 Gew.-% der Zusammensetzung vorhanden ist;
wobei das Saccharid mit hoher Formbarkeit eine Härte von 19,6 N (2 kp) oder mehr zeigt, wenn 150 mg des Saccharids unter Verwendung eines Stempels von 8 mm im Durchmesser unter einem Druck von 10 bis 50 kg/cm² zu einer Tablette geformt werden;
wobei das Saccharid mit geringer Formbarkeit eine Härte von weniger als 19,6 N (2 kp) zeigt, wenn 150 mg des Saccharids unter Verwendung eines Stempels von 8 mm im Durchmesser unter einem Druck von 10 bis 50 kg/cm² zu einer Tablette geformt werden;
wobei die schnell zergehende pharmazeutische Zusammensetzung innerhalb von 30 bis 200 Sekunden in einer Standardzerfallsprüfung in vitro, durchgeführt gemäß der US Pharmakopöe 24 (2000), Test Nr. 701, zerfällt.

2. Verfahren nach Anspruch 1, wobei die Stufe (b) vor oder simultan mit der Stufe (a) erfolgt.

3. Verfahren nach Anspruch 1, wobei die Feuchtgranulierungsstufe Wirbelschichtgranulierung umfasst.

4. Verfahren nach Anspruch 1, wobei der fünf- bis sechsgliedrige Ring ausgewählt ist aus Cyclopentenon-, Furanon-, Methylpyrazol-, Isoxazol- und Pyridinringen, substituiert an nicht mehr als einer Position.

5. Verfahren nach Anspruch 1, wobei der selektive Cyclooxygenase-2-inhibierende Wirkstoff ausgewählt ist aus Celecoxib, Deracoxib, Valdecoxib, Rofecoxib, Etoricoxib, 2-(3,5-Difluorphenyl)-3-[4-(methylsulfonyl)phenyl]-2-cyclopenten-1-on, (S)-6,8-Dichlor-2-(trifluormethyl)-2H-1-benzopyran-3-carbonsäure und 2-(3,4-Difluorphenyl)-4-(3-hydroxy-3-methyl-1-butoxy)-5-[4-(methylsulfonyl)phenyl]-3-(2H)-pyridazinon.

6. Verfahren nach Anspruch 1, wobei der selektive Cyclooxygenase-2-inhibierende Wirkstoff Celecoxib ist.

7. Verfahren nach Anspruch 1, wobei der selektive Cyclooxygenase-2-inhibierende Wirkstoff Valdecoxib ist.

8. Verfahren nach Anspruch 1, wobei das Saccharid mit geringer Formbarkeit ausgewählt ist aus Lactose, Mannit, Glucose, Saccharose und Xylit.

9. Verfahren nach Anspruch 1, wobei das Saccharid mit geringer Formbarkeit Mannit in Pulverqualität ist.

10. Verfahren nach Anspruch 1, wobei das Saccharid mit hoher Formbarkeit ausgewählt ist aus Maltose, Maltit, Sorbit und Oligosacchariden mit 2 bis 6 Monosaccharidresten.

11. Verfahren nach Anspruch 1, wobei das Saccharid mit hoher Formbarkeit Maltose ist.

12. Verfahren nach Anspruch 1, wobei das Benetzungsmittel in einer Gesamtmenge von 0,05 Gew.-% bis 5 Gew.-% der Zusammensetzung zugegeben wird.

13. Verfahren nach Anspruch 1, weiterhin umfassend die Zugabe eines Gleitmittels.

14. Verfahren nach Anspruch 13, wobei das Gleitmittel Siliziumdioxid ist.

15. Verfahren nach Anspruch 13, wobei das Gleitmittel in einer Gesamtmenge von 0,05 Gew.-% bis 5 Gew.-% der Zusammensetzung zugegeben wird.

16. Verfahren nach Anspruch 1, wobei der selektive Cyclooxygenase-2-inhibierende Wirkstoff in einer Gesamtmenge von 45 Gew.-% bis 75 Gew.-% der Zusammensetzung vorhanden ist.

17. Verfahren nach Anspruch 1, wobei das Saccharid mit hoher Formbarkeit in einer Gesamtmenge von 1 Gew.-% bis 10 Gew.-% der Zusammensetzung vorhanden ist.

18. Verfahren nach Anspruch 1, wobei das Saccharid mit geringer Formbarkeit in einer Gesamtmenge von 10 Gew.-% bis 90 Gew.-% der Zusammensetzung vorhanden ist.

19. Verfahren nach Anspruch 1, wobei das Gewichtsverhältnis des Saccharids mit hoher Formbarkeit zu dem Saccharid mit geringer Formbarkeit 2:100 bis 20:100 beträgt.

20. Verfahren nach Anspruch 1, weiterhin umfassend
(c) eine Stufe des Mischens der Granulate mit mindestens einem Schmiermittel, einem Süßungsmittel und einem Aromagebenden Mittel, um eine Tablettiermischung zu bilden, und
(d) eine Stufe des Verpressens der Tablettiermischung, um orale schnell zergehende Tabletten zu bilden.

21. Verfahren nach Anspruch 20, wobei die Parameter in der Stufe des Verpressens (d) so eingestellt sind, dass Tabletten mit einer Härte von 9,8 bis 98 N (1 bis 10 kp) bereitgestellt werden.

22. Verfahren nach Anspruch 1, wobei Stufe (b) eine Stufe des Mischens eines Saccharids mit geringer Formbarkeit und eines Gleitmittels mit dem Wirkstoff ist.

23. Orale schnell zergehende pharmazeutische Zusammensetzung, hergestellt durch das Verfahren nach einem der Ansprüche 1 bis 22.

24. Orale schnell zergehende Zusammensetzung nach Anspruch 23, umfassend einen selektiven Cyclooxygenase-2-inhibierenden Wirkstoff, dispergiert in einer Matrix, umfassend ein Saccharid mit geringer Formbarkeit und ein Saccharid mit hoher Formbarkeit.

25. Zusammensetzung nach Anspruch 24, weiterhin umfassend ein Benetzungsmittel.

26. Zusammensetzung nach Anspruch 25, wobei das Benetzungsmittel wie in Anspruch 1 definiert ist.

27. Zusammensetzung nach Anspruch 24, weiterhin umfassend ein Gleitmittel.

28. Zusammensetzung nach Anspruch 27, wobei das Gleitmittel wie in Anspruch 14 oder 15 definiert ist.

29. Zusammensetzung nach Anspruch 24, wobei der selektive Cyclooxygenase-2-inhibierende Wirkstoff in einer Menge von 1 Gew.-% bis 75 Gew.-% der Zusammensetzung vorhanden ist.

30. Zusammensetzung nach Anspruch 24, wobei der selektive Cyclooxygenase-2-inhibierende Wirkstoff in einer Menge von 45 Gew.-% bis 75 Gew.-% der Zusammensetzung vorhanden ist.

31. Zusammensetzung nach Anspruch 24, wobei das Saccharid mit geringer Formbarkeit wie in den Ansprüchen 8 und 9 definiert ist.

32. Zusammensetzung nach Anspruch 24, wobei das Saccharid mit geringer Formbarkeit in einer Menge von 10 Gew.-% bis 90 Gew.-% der Zusammensetzung vorhanden ist.

33. Zusammensetzung nach Anspruch 24, wobei das Saccharid mit hoher Formbarkeit wie in den Ansprüchen 10 und 11 definiert ist.

34. Zusammensetzung nach Anspruch 24, wobei das Saccharid mit hoher Formbarkeit in einer Menge von 1 Gew.-% bis 10 Gew.-% der Zusammensetzung vorhanden ist.

35. Zusammensetzung nach Anspruch 24, wobei das Gewichtsverhältnis des Saccharids mit hoher Formbarkeit zu dem Saccharid mit geringer Formbarkeit 2:100 bis 20:100 beträgt.

36. Zusammensetzung nach Anspruch 24, die eine Tablette ist.

37. Tablette nach Anspruch 36, die innerhalb von 30 bis 300 Sekunden in einer Standardzerfallsprüfung in vitro zerfällt.

38. Zusammensetzung nach Anspruch 36, die innerhalb von 5 bis 60 Sekunden nach dem Platzieren in der Mundhöhle eines Subjektes zerfällt.

39. Verwendung einer Zusammensetzung nach den Ansprüchen 24 bis 38 zur Herstellung eines Medikaments zur oralen Behandlung eines medizinischen Zustands oder einer medizinischen Störung bei einem Säugersubjekt, ausgewählt aus der Gruppe, bestehend aus Entzündung, Schmerz, Fieber, Arthritis, arthritischen Störungen, Asthma, Bronchitis, Menstruationskrämpfen, Frühgeburt, Tendinitis, Bursitis, allergischer Neuritis, Cytomegalovirus-Infektiosität, Apoptose, einschließlich HIVinduzierter Apoptose, Lumbago, Lebererkrankung, Psoriasis, Ekzem, Akne, Verbrennungen, Dermatitis und Schädigung durch ultraviolette Strahlung, postoperativer Entzündung, gastrointestinaler Zustände, Migränekopfschmerzen, Periarteriitis nodosa, Thyreoiditis, aplastischer Anämie, Hodgkinscher Krankheit, Skleroderma, rheumatischem Fieber, Diabetes Typ I, Erkrankung der motorischen Endplatte, Erkrankung der Substantia alba, Sarkoidose, nephrotischem Syndrom, Behcet-Syndrom, Polymyositis, Gingivitis, Nephritis, Hypersensibilität, Schwellung nach Verletzung, Myokardischämie, ophthalmischen Erkrankungen, akuter Verletzung des Augengewebes, Lungenentzündung, Osteoporose, Störungen des zentralen Nervensystems, allergischer Rhinitis, Atemnotsyndrom, Endotoxinschocksyndrom, kardiovaskulären Störungen mit Entzündungsbezug, vaskulären Erkrankungen, Koronararterienerkrankung, Aneurisma, vaskulärer Abstoßung, Arteriosklerose, Myokardinfarkt, Embolie, Schlaganfall, Thrombose, Angina, Koronarplaque-Entzündung, Störungen mit Angiogenesebezug, Neoplasie, Hornhauttransplantatabstoßung, Augenneovaskularisierung, Netzhautneovaskularisierung, Makuladegeneration, retrolentaler Fibroplasie, Glaukom, Magengeschwür, Hämangiomen, Störungen des weiblichen Reproduktionssystems, Fibrose, die bei Strahlentherapie auftritt und adenomatösen Polypen.

40. Verwendung nach Anspruch 39, wobei das Säugersubjekt ein menschliches Subjekt ist.

41. Verwendung nach Anspruch 40, die weiterhin eine Kombinationstherapie mit einem oder mehreren Wirkstoffen umfasst, ausgewählt aus Opioiden und anderen Analgetika.

42. Verwendung nach Anspruch 40, die weiterhin eine Kombinationstherapie mit einer Opioidverbindung umfasst, ausgewählt aus Codein, Meperidin und Morphin.

## Revendications

1. Procédé de préparation d'une composition pharmaceutique orale à fonte rapide, le procédé comprenant :
(a) une étape de granulation par voie humide d'un médicament inhibiteur sélectif de cyclooxygénase-2 avec un agent liant liquide comprenant un saccharide ayant une aptitude au moulage élevée, et
(b) une étape de mélange avec le médicament d'un saccharide ayant une faible aptitude au moulage, dans lequel lesdites étapes (a) et (b) ont lieu selon un quelconque ordre ou
simultanément pour aboutir à la formation de granules ;
où le procédé comprend un moyen pour inhiber l'agglomération du médicament, ledit moyen étant choisi dans le groupe consistant en addition d'un agent mouillant, un moyen pour pré-mouiller le matériau à granuler, et un moyen pour augmenter le débit d'air le long de la périphérie du bol de granulation ; où ledit agent mouillant est choisi dans le groupe consistant en tensioactifs, polymères et argiles hydrophiles ; où ledit tensioactif est choisi dans le groupe consistant en composés ammonium quaternaire, dioctyl sulfosuccinate de sodium, polyoxyéthylène alkylphényl éthers, copolymères blocs de polyoxyéthylène, copolymères blocs de polyoxypropylène, glycérides d'acides gras et de polyoxyéthylène, huiles d'acides gras de polyoxyéthylène, polyoxyéthylène alkyl éthers, esters d'acide gras de polyoxyéthylène, esters de polyoxyéthylène sorbitane, esters d'acides gras de propylène glycol, laurylsulfate de sodium, acides gras, sels d'acides gras, esters d'acides gras et de glycéryle, esters de sorbitan, tyloxapol et leurs mélanges ;
où le médicament inhibiteur sélectif de cyclooxygénase-2 est un composé ayant la formule : 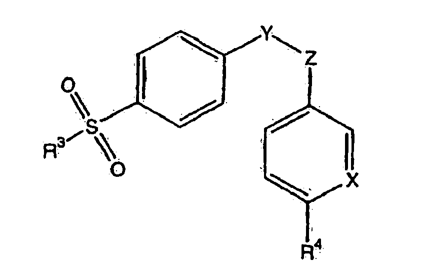 dans laquelle R³ est un groupe méthyle ou amino, R⁴ est un atome d'hydrogène ou un groupe alkyle ou alcoxy en C₁₋₄, X est N ou CR⁵ où R⁵ est un atome d'hydrogène ou d'halogène, et Y et Z sont indépendamment des atomes de carbone ou d'azote définissant des atomes adjacents d'un noyau à cinq ou six chaînons qui est non substitué ou substitué au niveau d'une ou de plusieurs positions par des groupes oxo, halo, méthyle ou halométhyle ;
où ledit médicament inhibiteur sélectif de cyclooxygénase-2 est présent en une quantité de 15% à 75% en poids de la composition ;
où le saccharide ayant une aptitude au moulage élevée présente une dureté de 19,6 N (2 kp) ou plus lorsque 150 mg du saccharide sont façonnés en comprimé avec un poinçon de 8 mm de diamètre sous une pression de 10 à 50 kg/cm² ;
où le saccharide ayant une faible aptitude au moulage présente une dureté inférieure à 19,6 N (2 kp) lorsque 150 mg du saccharide sont façonnés en comprimé avec un poinçon de 8 mm de diamètre sous une pression de 10 à 50 kg/cm² ; et
où la composition pharmaceutique à fonte rapide se désagrège en 30 à 200 secondes dans un essai de désagrégation *in vitro* standard conduit selon la norme U.S. Pharmacopeia 24 (2000), Test No. 701.

2. Procédé selon la revendication 1, dans lequel ladite étape (b) a lieu avant ou en même temps que ladite étape (a).

3. Procédé selon la revendication 1, dans lequel ladite étape de granulation par voie humide comprend une granulation en lit fluide.

4. Procédé selon la revendication 1, dans lequel le noyau à cinq ou six chaînons est choisi parmi les noyaux cyclopenténone, furanone, méthylpyrazole, isoxazole et pyridine substitués en au plus une position.

5. Procédé selon la revendication 1, dans lequel le médicament inhibiteur sélectif de cyclooxygénase-2 est choisi parmi le célécoxib, le déracoxib, le valdécoxib, le rofécoxib, l'étoricoxib, la 2-(3,5-difluorophényl)-3-[4-(méthylsulfonyl)phényl]-2-cyclopentèn-1-one, l'acide (S)-6,8-dichloro-2-(trifluorométhyl)-2H-1-benzopyran-3-carboxylique et la 2-(3,4-difluorophényl)-4-(3-hydroxy-3-méthyl-1-butoxy)-5-[4-(méthylsulfonyl)phényl]-3-(2H)-pyridazinone.

6. Procédé selon la revendication 1, dans lequel le médicament inhibiteur sélectif de cyclooxygénase-2 est le célécoxib.

7. Procédé selon la revendication 1, dans lequel le médicament inhibiteur sélectif de cyclooxygénase-2 est le valdécoxib.

8. Procédé selon la revendication 1, dans lequel ledit saccharide ayant une faible aptitude au moulage est choisi parmi le lactose, le mannitol, le glucose, le sucrose et le xylitol.

9. Procédé selon la revendication 1, dans lequel ledit saccharide ayant une faible aptitude au moulage est le mannitol de qualité poudre.

10. Procédé selon la revendication 1, dans lequel ledit saccharide ayant une aptitude au moulage élevée est choisi parmi le maltose, le maltitol, le sorbitol et des oligosaccharides ayant 2 à 6 résidus monosaccharide.

11. Procédé selon la revendication 1, dans lequel ledit saccharide ayant une aptitude au moulage élevée est le maltose.

12. Procédé selon la revendication 1, dans lequel ledit agent mouillant est ajouté en une quantité totale de 0,05 à 5% en poids de la composition.

13. Procédé selon la revendication 1, comprenant de plus l'addition d'un agent de glissement.

14. Procédé selon la revendication 13, dans lequel ledit agent de glissement est du dioxyde de silicium.

15. Procédé selon la revendication 13, dans lequel ledit agent de glissement est ajouté en une quantité totale de 0,05% à 5% en poids de la composition.

16. Procédé selon la revendication 1, dans lequel ledit médicament inhibiteur sélectif de cyclooxygénase-2 est présent en une quantité totale de 45% à 75% en poids de la composition.

17. Procédé selon la revendication 1, dans lequel ledit saccharide ayant une aptitude au moulage élevée est présent en une quantité totale de 1% à 10% en poids de la composition.

18. Procédé selon la revendication 1, dans lequel ledit saccharide ayant une faible aptitude au moulage est présent en une quantité totale de 10% à 90% en poids de la composition.

19. Procédé selon la revendication 1, dans lequel le rapport dudit saccharide ayant une aptitude au moulage élevée audit saccharide ayant une faible aptitude au moulage est de 2 :100 à 20 :100.

20. Procédé selon la revendication 1, comprenant de plus :
(c) une étape de mélange desdits granules avec au moins un agent parmi un lubrifiant, un agent édulcorant et un agent aromatisant pour former un mélange d'empastillage, et
(d) une étape de compression du mélange d'empastillage pour former des comprimés à usage oral à fonte rapide.

21. Procédé selon la revendication 20, dans lequel les paramètres sont fixés dans ladite étape de compression (d) de façon à fournir des comprimés ayant une dureté de 9,8 à 98 N (1 à 10 kp).

22. Procédé selon la revendication 1, dans lequel l'étape (b) est une étape de mélange avec le médicament d'un saccharide ayant une faible aptitude au moulage et d'un agent de glissement.

23. Composition pharmaceutique à usage oral à fonte rapide préparée par le procédé selon l'une quelconque des revendications 1 à 22.

24. Composition à usage oral à fonte rapide selon la revendication 23, comprenant un médicament inhibiteur sélectif de cyclooxygénase-2 dispersé dans une matrice comprenant un saccharide de faible aptitude au moulage et un saccharide ayant une aptitude au moulage élevée.

25. Composition selon la revendication 24 comprenant de plus un agent mouillant.

26. Composition selon la revendication 25, dans laquelle ledit agent mouillant est tel que défini dans la revendication 1.

27. Composition selon la revendication 24, comprenant de plus un agent de glissement.

28. Composition selon la revendication 27, dans laquelle ledit agent de glissement est tel que défini dans la revendication 14 ou 15.

29. Composition selon la revendication 24, dans laquelle le médicament inhibiteur sélectif de cyclooxygénase-2 est présent en une quantité de 1% à 75% en poids de la composition.

30. Composition selon la revendication 24, dans laquelle le médicament inhibiteur sélectif de cyclooxygénase-2 est présent en une quantité de 45% à 75% en poids de la composition.

31. Composition selon la revendication 24, dans laquelle ledit saccharide ayant une faible aptitude au moulage est tel que défini dans les revendications 8 et 9.

32. Composition selon la revendication 24, dans laquelle ledit saccharide ayant une faible aptitude au moulage est présent en une quantité de 10% à 90% en poids de la composition.

33. Composition selon la revendication 24, dans laquelle ledit saccharide ayant une aptitude au moulage élevée est tel que défini dans les revendications 10 et 11.

34. Composition selon la revendication 24, dans laquelle ledit saccharide ayant une aptitude au moulage élevée est présent en une quantité de 1 % à 10% en poids de la composition.

35. Composition selon la revendication 24, dans laquelle le rapport en poids dudit saccharide ayant une aptitude au moulage élevé audit saccharide ayant une faible aptitude au moulage est de 2 :100 à 20 :100.

36. Composition selon la revendication 24 qui est un comprimé.

37. Comprimé selon la revendication 36 qui se désagrège en 30 à 300 secondes dans un essai standard de désagrégation *in vitro.*

38. Composition selon la revendication 36 qui se désagrège en 5 à 60 secondes après mise en place dans la cavité orale d'un sujet.

39. Utilisation d'une composition selon les revendications 24 à 38 pour la préparation d'un médicament destiné au traitement oral d'un état ou d'un trouble médical chez un sujet mammifère, choisi dans le groupe consistant en inflammation, douleur, fièvre, arthrite, troubles arthritiques, asthme, bronchite, douleurs menstruelles, travail avant terme, tendinite, bursite, névrite allergique, infection à cytomégalovirus, apoptose incluant une apoptose induite par le VIH, lumbago, affection du foie, psoriasis, eczéma, acné, brûlures, dermatite et lésion par rayonnement ultraviolet, inflammation post-opératoire, états gastro-intestinaux, maux de tête migraineux, périartérite noueuse (PAN), thyroïdite, anémie aplasique, maladie de Hodgkin, scléroedème, fièvre rhumatismale, diabète de type I, affection des articulations neuromusculaires, maladie de la matière blanche, sarcoïdose, syndrome néphrotique, syndrome de Behcet, polymyosite, gingivite, néphrite, hypersensibilité, gonflement consécutif à une lésion, ischémie du myocarde, maladies ophtalmiques, lésion aiguë du tissu oculaire, inflammation pulmonaire, ostéoporose, troubles du système nerveux central, rhinite allergique, syndrome de détresse respiratoire, syndrome du choc d'endotoxine, troubles cardiovasculaires associés à une inflammation, maladies vasculaires, affection des artères coronaires, anévrisme, rejet vasculaire, artériosclérose, athérosclérose, infarctus du myocarde, embolie, accident vasculaire cérébral, thrombose, angine, inflammation de la plaque coronarienne, troubles associés à l'angiogenèse, néoplasie, rejet d'une greffe de cornée, néovascularisation oculaire, néovascularisation rétinienne, dégénérescence maculaire, fibroplasie retrocristallinienne, glaucome, ulcère gastrique, hémangiomes, troubles du système reproducteur féminin, fibrose qui apparaît avec une thérapie par radiation et polypes adénomateux.

40. Utilisation selon la revendication 39, dans laquelle ledit sujet mammifère est un sujet humain.

41. Utilisation selon la revendication 40 qui comprend de plus une thérapie de combinaison avec un ou plusieurs médicaments choisis parmi les opioïdes et d'autres analgésiques.

42. Utilisation selon la revendication 40 qui comprend de plus une thérapie de combinaison avec un composé opioïde choisi parmi la codéine, la mépéridine et la morphine.
